(19) Europäisches Patentamt
European Patent Office
Office européen des brevets

(11) **EP 0 740 801 B1**

(12) **EUROPÄISCHE PATENTSCHRIFT**

(45) Veröffentlichungstag und Bekanntmachung des Hinweises auf die Patenterteilung:
**03.05.2000 Patentblatt 2000/18**

(51) Int. Cl.[7]: **G01S 13/87**, G01S 13/82, G01S 7/00, A61B 5/00

(21) Anmeldenummer: **94926055.8**

(22) Anmeldetag: **16.09.1994**

(86) Internationale Anmeldenummer:
**PCT/AT94/00131**

(87) Internationale Veröffentlichungsnummer:
**WO 95/08127 (23.03.1995 Gazette 1995/13)**

(54) **POSITIONSMESSVORRICHTUNG UND VERFAHREN ZUM FESTSTELLEN DER LAUFZEIT EINER ABGESANDTEN ENERGIESTRAHLUNG ZWISCHEN EINEM BASISSTANDORT UND EINEM BEWEGLICHEN OBJEKT**

POSITION MEASURING DEVICE AND PROCESS FOR FINDING THE TIME OF FLIGHT OF RADIATED ENERGY BETWEEN A BASE AND A MOVING OBJECT

DISPOSITIF DE MESURE DE POSITION ET PROCEDE PERMETTANT DE DETERMINER LE TEMPS DE PROPAGATION D'UN RAYONNEMENT ENERGETIQUE EMIS ENTRE UN POSTE DE BASE ET UN OBJET MOBILE

(84) Benannte Vertragsstaaten:
**CH DE DK ES FR GB GR IT LI NL SE**

(30) Priorität: **16.09.1993 AT 187393**

(43) Veröffentlichungstag der Anmeldung:
**06.11.1996 Patentblatt 1996/45**

(73) Patentinhaber:
• **Riener, Karl Stefan**
  **4563 Micheldorf (AT)**
• **Niederndorfer, Friedrich, Dipl.-Ing.**
  **4861 Aurach am Hongar (AT)**
• **Leitner, Rainer, Mag.**
  **4861 Schörfling am Attersee (AT)**

(72) Erfinder:
• **Riener, Karl Stefan**
  **4563 Micheldorf (AT)**

• **Niederndorfer, Friedrich, Dipl.-Ing.**
  **4861 Aurach am Hongar (AT)**
• **Leitner, Rainer, Mag.**
  **4861 Schörfling am Attersee (AT)**

(74) Vertreter:
**Secklehner, Günter, Dr.**
**Rechtsanwalt,**
**Pyhrnstrasse 1**
**8940 Liezen (AT)**

(56) Entgegenhaltungen:
GB-A- 2 207 787          US-A- 4 700 306

• **STEVENS 'Secondary surveillance radar' 1988 , ARTECH HOUSE INC , NORWOOD, MA, US siehe Seite 251, Zeile 1 - Seite 257, Zeile 2**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zum Feststellen der Laufzeit einer abgesandten Energiestrahlung zwischen einem Basisstandort und zumindest einem davon distanzierten, beweglichen Objekt, wie es im Oberbegriff des Patentanspruches 1 beschrieben ist.

Aus der US 4 700 306 A ist ein Positionsmeßsystem zum Darstellen der Position von Schiffen auf einem Monitor bekannt, das von einer Fernsehstation mit Signalen versorgt wird. Das Verfahren zum Bestimmen der Position benötigt zwei Meßstationen, insbesondere einen Master und einen Slave, wobei vom Master eine Energiesendestrahlung ausgestrahlt wird, worauf von einer Sende- und Empfangseinheit im Positionsmelder, der sich am Schild befindet, die Energiesendestrahlung empfangen wird. Beim Eingang der Energiesendestrahlung am Empfänger wird der Sender des Positionsmelders zur Absendung einer Energierücksendestrahlung aktiviert. Die Energierücksendestrahlung wird von der Master- und der Slavestation empfangen, wobei die Energierücksendestrahlung, die am Slave eintrifft, vom Slave anschließend an den Master weitergeleitet wird. Durch die unterschiedlich langen Laufzeiten der beiden Signale, also dem direkten und indirekten Signal, kann die Position des Schiffes über eine Dreiecksfunktion berechnet werden. Nachteilig ist hierbei, daß erst beim Aussenden der Energiesendestrahlung der Positionsmelder aktiviert wird, sodaß eine relativ hohe Zeitverzögerung vom Empfang der Energiesendestrahlung bis zur Aussendung der Energierücksendestrahlung gegeben ist und somit die Zeitdauer für zwei Abfragezyklen hintereinander verlängert wird.

Weiters ist aus der GB 2 207 787 A1 ein Positionsbestimmungssystem für verlorengegangene Sachen, insbesondere für Fahrzeuge, bekannt. In diesem Verfahren wird über ein Radiosignal, das von Radiosendern ausgesendet wird, die Position der verlorenen Sache bestimmt. Dabei sendet die verlorene Sache, insbesondere das Fahrzeug, eine Energierücksendestrahlung aus, die anschließend von den Radiosendern an eine Zentralstation übersandt wird und von dieser die Position der verlorengegangenen Sache, insbesondere des Fahrzeuges, berechnet wird. Nachteilig ist hierbei, daß die Zeitdauer zum wiederholten Aussenden eines Meßzyklusses sehr hoch ist, da die Zeitdauer, die zum Übersenden der Sendestrahlung vom am weitesten entfernten Radiosender zur Zentraleinheit abgewartet werden muß.

Es sind bereits Verfahren zum Feststellen der Laufzeit einer abgesandten Energiestrahlung zwischen einem Basisstandort und einem Objekt bekannt - gemäß DE 35 26 564 A1 -, bei dem eine gerichtete, mit einer Winkelcodierung versehene Energiesendestrahlung von einem Basisstandort und einem Nebenstandort ausgesendet wird. Die einlangende Energiesendestrahlung wird am Objekt erfaßt, und aus der übermittelten Winkel-codierung wird die Position des Objektes auf einer Fläche errechnet. Nach Feststellen der Position können diese Positionsdaten bevorzugt über drahtlose Übertragungsvorrichtungen an eine Zentrale übermittelt werden. Nachteilig ist hierbei, daß zwei Standorte zum Absenden einer Energiesendestrahlung benötigt werden und außerdem jeweils die Winkelstellung der Energiesendestrahlung mitübertragen werden muß und die gesamte Auswertung der Position am beweglichen Objekt stattfindet.

Der vorliegenden Erfindung liegt die Aufgabe zugrunde, ein Verfahren zu schaffen, mit dem es möglich ist, ständig die Position von einem oder mehreren Objekten zu überwachen und dabei den technischen Aufwand im Bereich des Positionsmelders so gering wie möglich zu halten.

Diese Aufgabe der Erfindung wird durch die Merkmale im Kennzeichenteil des Patentanspruches 1 gelöst. Durch die Verwendung einer Energiesende- und einer Energierücksendestrahlung kann der Positionsmelder sehr einfach aufgebaut sein, da er lediglich das Einlangen der Energiesendestrahlung erkennen und dementsprechend eine Energierücksendestrahlung absenden muß. Damit kann er räumlich sehr klein und auch leicht gebaut werden, sodaß er sich bevorzugt für die Anwendung bei der Positionsfeststellung von Personen bzw. der Bewegung von Maschinenteilen und dgl. eignet. Weiters wird durch diese Vorgangsweise eine flächendeckende Überwachung von mehreren auf einer Fläche wahllos verteilten Objekten aufeinanderfolgend ermöglicht und, es ist des weiteren möglich, jedes einzelne Objekt nach einem vorbestimmten Rythmus bzw. bei mehreren Objekten diese nach jeweils gewünschten Kriterien aufeinanderfolgend hinsichtlich ihrer Position abzufragen. Ein überraschender und nicht vorhersehbarer Vorteil dieser Lösung liegt des weiteren aber auch darin, daß damit eine Echtzeiterfassung der Bewegungen des Objektes möglich ist und damit in Echtzeit die Objektbewegungen auch außerhalb der zu überwachenden Fläche dargestellt werden können. Dazu kommt, daß ein weiterer überraschender Vorteil dieser Lösung darin liegt, daß die Genauigkeit der errechneten Position sehr hoch ist, da bei Verwendung eines Richtstrahles oder bei Verwendung von zwei Energiesendestrahlungen, die von einem Basisstandort und einem Nebenstandort abgesendet werden, sich lediglich Positionsdifferenzen bei sich bewegenden Objekten durch die unterschiedliche Entfernung des Objektes zum Basisstandort und Nebenstandort ergeben können, wodurch die Ermittlung der Position der Objekte auch bei höheren Eigengeschwindigkeiten der Objekte einwandfrei möglich ist und somit auch eine exakte Ermittlung der Geschwindigkeit und gegebenenfalls der Beschleunigung sowie der von den Objekten zurückgelegte Weg, falls gewünscht auch in Echtzeit, ermittelt werden kann.

Vorteilhaft ist auch ein Vorgehen nach Patentanspruch 2, da dadurch mit der Aussendung einer Energiesendestrahlung nur von einem Basisstandort das Auslangen gefunden werden kann, jedoch durch die Verwendung einer Energierücksendestrahlung, die Position des Objektes am Basisstandort festgestellt werden kann, ohne daß eine Aussendung einer Energiesendestrahlung von einem weiteren Standort notwendig ist. Dadurch wird auch ein Positionsfehler bei der Berechnung der Position des Objektes, der dadurch entstehen könnte, daß zwei gerichtete Energiesendestrahlungen zu unterschiedlichen Zeitpunkten am Objekt aufgrund unterschiedlicher Scannrichtung oder dgl. eintreffen, vermindert bzw. überhaupt ausgeschaltet.

Vorteilhaft ist aber auch ein Vorgehen nach Patentanspruch 3, wodurch in einfacher Weise über die ermittelte Zeitdauer die Distanz zwischen dem Basisstandort, von welchem die Energiesendestrahlung abgesandt wurde und dem Objekt feststeht und mit der gleichzeitigen Zuordnung des Winkels des Richtstrahls die Position auf der überwachten Fläche einfach ermittelt werden kann.

Durch den Verfahrensablauf nach Patentanspruch 4 wird ein gleichmäßiger Scannvorgang über die gesamte Fläche ermöglicht, wobei eine mechanische oder steuerungstechnische Synchronisierung für Sendevorrichtungen oder dgl. wegfällt.

Es ist aber auch möglich, nach Patentanspruch 5 vorzugehen, wodurch mit nur einer Energiesendestrahlung durch die Auslösung von zwei Energierücksendestrahlungen ohne jeweiliger Festlegung der Ausstrahlrichtung der Energiesendestrahlung die exakte Position des Objektes errechnet werden kann und ein durch die unterschiedlichen Entfernungen des Objektes vom Basis- und Nebenstandort bedingten Meßfehler bei höheren Geschwindigkeiten bzw. Beschleunigungen des Objektes vermieden werden kann, da zum gleichen Zeitpunkt, nämlich dem Einlangen der Energiesendestrahlung die Energierücksendestrahlungen ausgelöst werden, die zur Laufzeit und somit zur Distanzberechnung verwendet werden.

Bei einem Vorgehen nach Patentanspruch 6 wird in vorteilhafter Weise erreicht, daß eine Echtzeitdarstellung mit geringstmöglichen Fehlertoleranzen möglich ist.

Vorteilhaft ist aber auch ein Verfahrensablauf nach Patentanspruch 7, da die Meßwertermittlung aufgrund der geringeren Anzahl von gleichzeitig anfallenden Rücksendestrahlungen vereinfacht ist und Signalüberlagerungen bzw. Fehlzuweisungen dadurch vermieden werden können.

Die Zuordnung der einlangenden Energierücksendestrahlung kann aber auch durch die Maßnahmen nach Patentanspruch 8 verbessert und vor allem eine Fehlauswertung ausgeschaltet werden.

Es sind aber auch die Verfahrensschritte nach Patentanspruch 9 von Vorteil, da jedem Start- und Stoppimpuls bzw. jeder steuerrelevanten Information eine Identifikation zugeordnet ist. Durch die Verwendung dieser Identifikationscodes ist es auch möglich, eine beliebige Mehrzahl von Objekten wahlweise gleichzeitig oder alternierend zur Positionsbestimmung anzusprechen.

Die Ermittlung der für die Berechnung der Position verwendeten Zeitsignale wird durch die Maßnahmen nach Patentanspruch 10 erheblich erleichtert. Dazu kommt, daß die Exaktheit der Meßwertermittlung durch die geringere Frequenz der Energierücksendestrahlung verbessert und insbesondere die Interpolation des Zeitsignals erleichtert wird.

Durch die weiteren Maßnahmen nach Patentanspruch 11 ist es möglich, die Ermittlung der Position von Objekten auch dann vorzunehmen, wenn der Basis- und/oder der Nebenstandort nicht in einer fixen gleichbleibenden Distanz angeordnet sind, wobei die Meßgenauigkeit bei der Ermittlung der Position des Objektes durch die Ermittlung und/oder Überwachung dieser bevorzugt veränderbaren Distanz mit derjenigen bei Verwendung ortsfester Basis- und/oder Nebenstandorte vergleichbar ist.

Durch das Vorgehen nach Patentanspruch 12 kann die Anzahl der zu ermittelnden Distanzen bzw. Entfernungen zur Berechnung der Position der Objekte verringert werden.

Vorteilhaft ist auch ein Ablauf gemäß Patentanspruch 13, da dadurch keine eigenen Energiesendestrahlungen bzw. Verbindungsmedien zwischen den Vermessungsvorrichtungen und den Positionsmeldern benötigt werden.

Durch das Vorgehen nach Patentanspruch 14 wird ein definitiver Startzeitpunkt für die Zeitfeststellung unabhängig von anderen Informationen festgelegt, wodurch Fehler bei der Meßwertermittlung verringert werden können.

Die Vorgangsweise nach Patentanspruch 15 ermöglicht in vorteilhafter Weise eine hohe Abfragedichte der Position der einzelnen Objekte durch die zeitüberlagerte Aussendung von Identifikationscodes sowie der sonstigen Impulse sowie Codes innerhalb eines maximalen Meßzyklusses, sodaß die Positionsermittlungen der einzelnen Objekte einander überlagern.

Vorteilhaft ist es bei der Anordnung von mehreren Positionsmeldern auf einem Objekt, wenn diese gemäß der Verfahrensweiterbildung in Patentanspruch 16 betrieben werden, da damit die Relativstellung der einzelnen Positionsmelder während einer Positionserfassung zusätzlich zum gleichen Zeitpunkt, also nahezu in Echtzeit, möglich ist.

Vorteilhaft sind auch die Maßnahmen nach Patentanspruch 17, da dadurch eine unmittelbare Feststellung der Bewegungsobjekte, beispielsweise auf einem Bildschirm ermöglicht wird, sodaß auf einem gewissen Bewegungsablauf der Objekte, beispielsweise die Stellung einzelner Spieler bei einem Mannschaftsspiel wie Fußball, Handball, Volleyball bzw. Cricket oder dem Bewegungsablauf beim Tennis oder Golfspielen unmittelbar durch den Trainer Einfluß genommen werden kann.

Durch die Verfahrensschritte nach Patentanspruch 18 ist es möglich, die erfindungsgemäße Positionsmeßeinrichtung auch zur Feststellung von bewegungsmotorischen Abläufen an Menschen bzw. Tieren zu verwenden.

Die Erfindung wird im nachfolgenden anhand der in den Zeichnungen dargestellten Ausführungsbeispiele näher erläutert.

Es zeigen:

Fig. 1 die Positionsmeßeinrichtung in schematischer, vereinfachter Darstellung in der Verwendung auf einer abgegrenzten Fläche, beispielsweise einem Sportplatz, in Draufsicht sowie ein zugehöriges Blockschaltbild in vereinfachter, schematischer Darstellung;

Fig. 2 die Anordnung und Ausbildung einer erfindungsgemäßen Positionsmeßeinrichtung auf einer abgegrenzten Fläche beispielsweise einem Lagerplatz in Draufsicht und vereinfachter, schematischer Darstellung;

Fig. 3 ein Diagramm mit dem Signalverlauf der Energiesende- und Energierücksendestrahlung der Positionsmeßeinrichtung nach Fig. 2 in zeitlichem Ablauf;

Fig. 4 die schematische Darstellung der Positionsmeßeinrichtung auf einem abgegrenzten Feld, beispielsweise einem Spielfeld mit unterschiedlichen aufeinander folgenden Positionen eines beweglichen Objektes und zur wahlweisen oder gleichzeitigen Verwendung auf einander benachbarten Flächen in Draufsicht und schematischer Darstellung;

Fig. 5 eine andere Ausführungsvariante des Signalverlaufs der Energiesende- und Energierücksendestrahlung der Positionsmeßeinrichtung nach Fig. 4 in zeitlichem Verlauf;

Fig. 6 die weitere Anordnung und Ausbildung einer erfindungsgemäßen Positionsmeßeinrichtung entlang einer Bahn, z.B. einer Laufbahn mit den darin eingetragenen, unterschiedlich aufeinander folgenden Positionen eines sich bewegenden Objektes in Draufsicht und schematischer Darstellung;

Fig. 7 die andere Anordnung und Ausbildung einer erfindungsgemäßen Positionsmeßeinrichtung für mehrere Objekte mit einer gerichteten Energiesendestrahlung;

Fig. 8 die Anordnung und Ausbildung einer erfindungsgemäßen Positionsmeßeinrichtung zur Ermittlung einer räumlichen Bewegung eines Positionsmelders mit mehreren auf unterschiedlichen Gliedmaßen einer Person angeordneten Positionsmeldern in vereinfachter, schematischer Darstellung;

Fig. 9 eine Ausführungsvariante für die Anordnung und Ausbildung einer erfindungsgemäßen Positionsmeßeinrichtung im Bereich einer Schanze für Schispringer in Seitenansicht und vereinfachter, schematischer Darstellung;

Fig. 10 Positionsmeßeinrichtung nach Fig. 9 in Draufsicht.

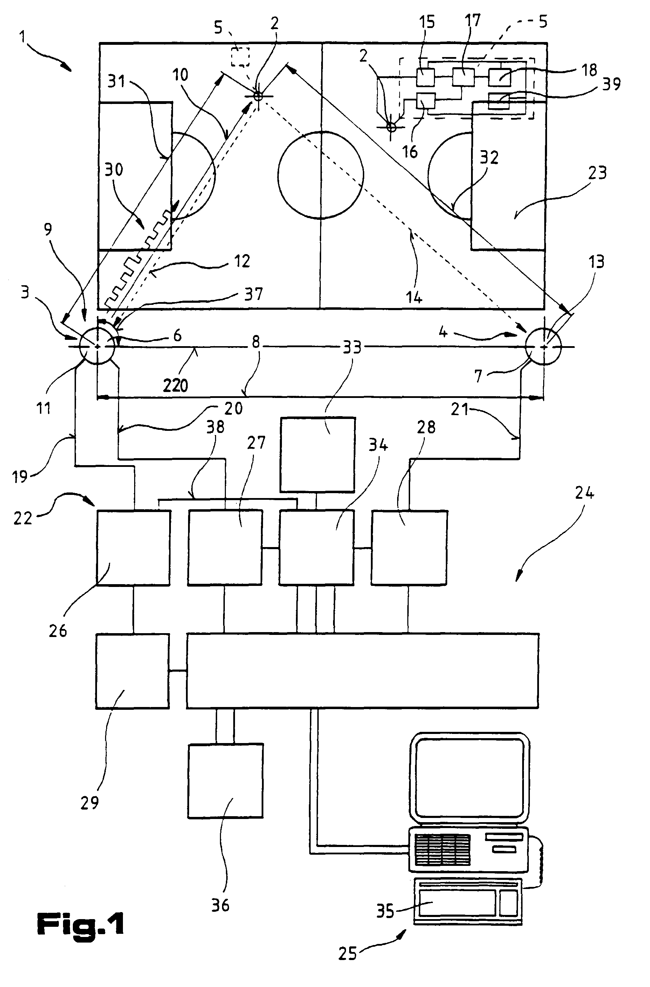

In Fig. 1 ist eine erfindungsgemäße Positionsmeßeinrichtung 1 für die Bestimmung einer Position eines Objektes 2 gezeigt. Die Positionsmeßeinrichtung 1 besteht dabei aus einer ersten Vermessungsvorrichtung 3, einer zweiten Vermessungsvorrichtung 4 und einem am Objekt 2 angeordneten Positionsmelder 5. Der Positionsmelder 5 ist gemeinsam mit dem Objekt 2 beweglich angeordnet, wogegen die Vermessungsvorrichtungen 3 und 4 stationär auf einem Basisstandort 6 und einem Nebenstandort 7 in einer fixen vorherbestimmbaren Distanz 8 voneinander angeordnet sind.

Die am Basisstandort 6 angeordnete Vermessungsvorrichtung 3 umfaßt einen Sender 9 zum Aussenden einer Energiesendestrahlung 10 und einen Empfänger 11 zum Empfangen einer Energierücksendestrahlung 12. Die am Nebenstandort 7 ange-ordnete Vermessungsvorrichtung 4, umfaßt dagegen im gezeigten Ausführungsbeispiel nur einen Empfänger 13, der eine Energierücksendestrahlung 14 aufnehmen kann. Der Positionsmelder 5 ist ebenfalls mit einem Empfänger 15 für die Energiesendestrahlung 10 und mit einem Sender 16 für die Energierücksendestrahlung 12 und 14 ausgestattet. Dem Empfänger 15 und/oder Sender 16 ist eine Decodiereinheit 17 zugeordnet, die ebenso wie Empfänger 15 und Sender 16 von einer Stromquelle 18 mit Energie versorgt werden.

Die Vermessungsvorrichtung 3 und 4 sind über Leitungen 19 bis 21 mit einer Steuereinrichtung 22 verbunden. Diese Verbindung kann aber auch drahtlos über entsprechend codierte Sende- und Empfangsanlagen erfolgen. Diese Steuereinrichtung 22 kann sich beispielsweise in einer Vermessungsvorrichtung 3 und/oder 4 befinden und ist ebenso wie diese außerhalb einer Fläche 23 angeordnet, innerhalb der sich die zu überwachenden Objekte 2 bewegen. Diese mit den Positionsmeldern 5 ausgestatteten Objekte 2 können beispielsweise Spieler einer Sportmannschaft, beispielsweise einer Fußballmannschaft sein, deren Leistung bzw. deren taktische Bewegungen am Spielfeld entweder in Echtzeit dargestellt oder in der Folge analysiert werden soll. Neben der Stellung der Objekte 2 kann selbstverständlich auch deren Geschwindigkeit zwischen zwei Standorten bzw. die Beschleunigung und andere daraus ableitbare Daten gewonnen werden.

Für die Aufnahme und Auswertung dieser Daten bzw. zur Feststellung einer Position eines Objektes 2 innerhalb der Fläche 23, also einer Spielfläche, ist die Steuereinrichtung 22, die durch eine Rechnereinheit 24 gebildet sein kann, dargestellt.

Selbstverständlich kann diese Rechnereinheit 24 entweder durch einen entsprechenden Personalcomputer in Industrieausführung ausgeführt sein, in dem die notwendigen zusätzlichen Steuerelemente auf einer Einschubkarte angeordnet sind, oder es wird dieser Personalcomputer nur als Eingabegerät 25 benutzt und die Ablaufsteuerung und Auswertung der in den Vermessungsvorrichtungen 3 und 4 der gewonnenen Daten erfolgt in einer selbstprogrammierbaren Steuerung oder einer speziell für diesen Anwendungsfall konfigurierten Rechnereinheit 24.

Zur Herstellung der Energiesendestrahlung 10 und der Auswertung der Energierücksendestrahlung 12 bzw. 14 ist die Rechnereinheit 24 mit einer Sendereinheit 26 bzw. zwei Empfängereinheiten 27 und 28 ausgestattet.

Weiters weist die Rechnereinheit 24 eine Codiereinheit 29 auf, mit welcher der Energiesendestrahlung 10 ein Identifikationscode 30 überlagert wird. Diese Überlagerung kann durch Amplituden- oder Frequenzmodulation oder sonstige andere technische Mittel erfolgen.

Dieser Identifikationscode 30 ermöglicht es, jeweils einzelne Positionsmelder 5 der Objekte 2 anzusprechen, sodaß die Positionsmelder 5 zur Feststellung ihrer Entfernung 31, 32 von den beiden Vermessungsvorrichtungen 3, 4 bzw. dem Basisstandort 6 und/oder dem Nebenstandort 7 durch die Energierücksendestrahlung 12, 14 an die Vermessungsvorrichtungen 3, 4 ermittelt werden kann. Die Entfernungen 31, 32 werden über das Zeitäquivalent der Laufzeit der Energiesendestrahlung 10 und der Energierücksendestrahlung 12 und 14 ermittelt.

Dazu ist in der Rechnereinheit 24 ein Taktgenerator 33, eine Zählereinheit 34 und eine entsprechende Ausbildung bzw. Softwareausstattung in der Rechnereinheit 24 vorgesehen.

Zum Feststellen der Position jedes Objektes 2 kann nun beispielsweise über ein Befehlseingabegerät 35, z.B. eine Tastatur, eine Bezeichnung des Objektes 2 eingegeben werden, dessen Position, Geschwindigkeit oder Beschleunigung ermittelt werden soll.

Über die Festlegung des Objektes 2 wird aus einem Speicher 36 der diesem Objekt 2 entsprechende Identifikationscode 30 abgefragt und über die Codiereinheit 29 dieser Identifikationscode 30 über die Sendereinheit 26 dem Sender 9 in der Vermessungsvorrichtung 3 zugeführt und von diesem ausgesandt. Bei dieser Energiesendestrahlung 10 kann es sich beispielsweise um eine elektromagnetische Wellenform handeln, die kreis-bzw. kugelförmig oder über einen bestimmten Ausstrahlungswinkel 37 abgestrahlt wird, der so groß ist, daß zumindest die Fläche 23 abgedeckt ist, je nachdem, ob eine Richtstrahl- oder eine Rundstrahlantenne oder dgl. verwendet wird. Für diese Absendung der Energiesendestrahlung 10 können alle aus dem Stand der Technik bekannten Verfahren und zugehörigen Antennen und Vorrichtungteile verwendet werden.

Gleichzeitig mit der Aktivierung der Sendereinheit 26 wird von der Sendereinheit 26 die Zählereinheit 34 über eine Steuerleitung 38, z.B. einem Startimpuls bzw. Startsignal, gestartet und zählt die von dem Taktgenerator 33 gelieferten Zeitimpulse. Langt nun die ausgestrahlte Energiesendestrahlung 10 im Empfänger 15 des Positionsmelders 5 ein, so wird über die Decodiereinheit 17 festgestellt, ob der im Energiesendestrahlung 10 enthaltene Identifikationscode 30 mit dem in einem Speicher 39 des Positionsmelders 5 hinterlegten Identifikationscode 30 übereinstimmt. Ist eine Übereinstimmung gegeben, so wird die Absendung der Energierücksendestrahlung 12 und 14 ausgelöst. Selbstverständlich ist es bei der Verwendung eines Rundstrahlers beim Positionsmelder 5 möglich, auch nur eine Energierücksendestrahlung 12 abzusenden.

Langt nunmehr die Energierücksendestrahlung 12 und gegebenenfalls 14 am jeweiligen Empfänger 11 bzw. 13 der Vermessungsvorrichtung 3 bzw. 4 ein, so wird über die Empfängereinheit 27 bzw. 28 ein Steuersignal an die Zählereinheit 34 abgegeben, welches beispielsweise bewirkt, daß der jeweilige Zählerstand in einem Speicher 36 der Rechnereinheit 24 bzw. in der Rechnereinheit 24 hinterlegt wird.

Aufgrund der zwischen dem Start und dem Stoppimpuls ermittelten Anzahl der vom Taktgenerator 33 abgegebenen Taktimpulse wird die Zeitdauer ermittelt, die die Energiesendestrahlung 10 von Sender 9 bis zum Positionsmelder 5 und von diesem die Energierücksendestrahlung 12 bzw. 14 zurück zu den Empfängern 11 und 13 der Vermessungsvorrichtungen 3 und 4 benötigt hat.

Die Vorgangsweise zum Ermitteln der Realzeiten und die daraus erfolgende Berechnung der Entfernungen 31, 32 sowie einen den der Geschwindigkeit. der Beschleunigung oder sonstiger Daten wird dann anhand der nachfolgenden Ausführungsbeispiele noch im Detail beschrieben.

Selbstverständlich ist es auch möglich, daß anstelle der Eingabe eines Kennzeichens für ein einzelnes Objekt 2 zur fortlaufenden Abfrage der Positionen einer Vielzahl von Objekten 2 ein entsprechendes Programm im Speicher 36 der Rechnereinheit 24 hinterlegt ist, sodaß zyklisch aufeinanderfolgend einzelne oder alle der zu überwachenden Objekte 2 abgefragt werden können.

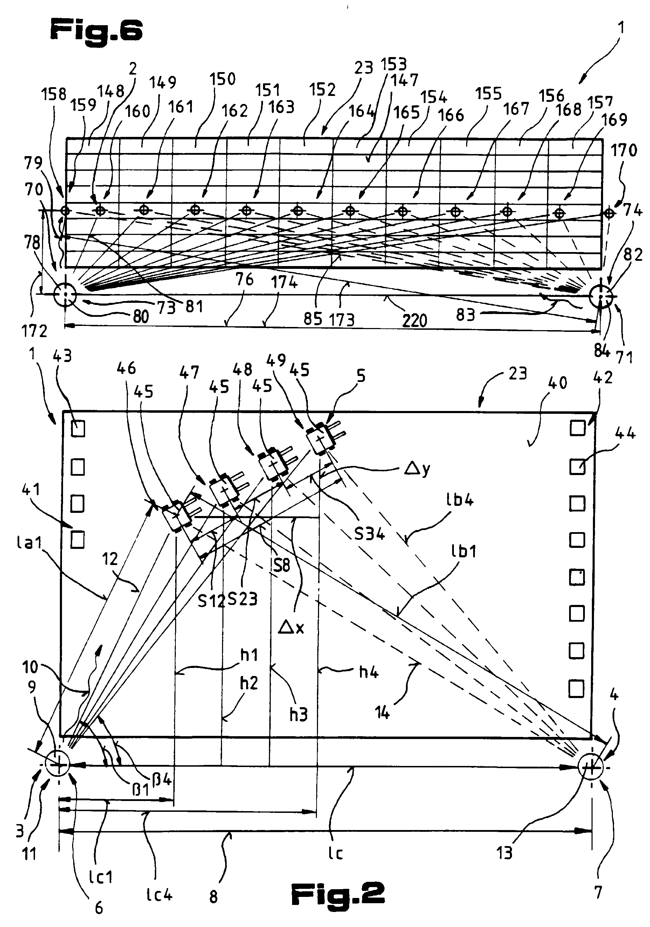

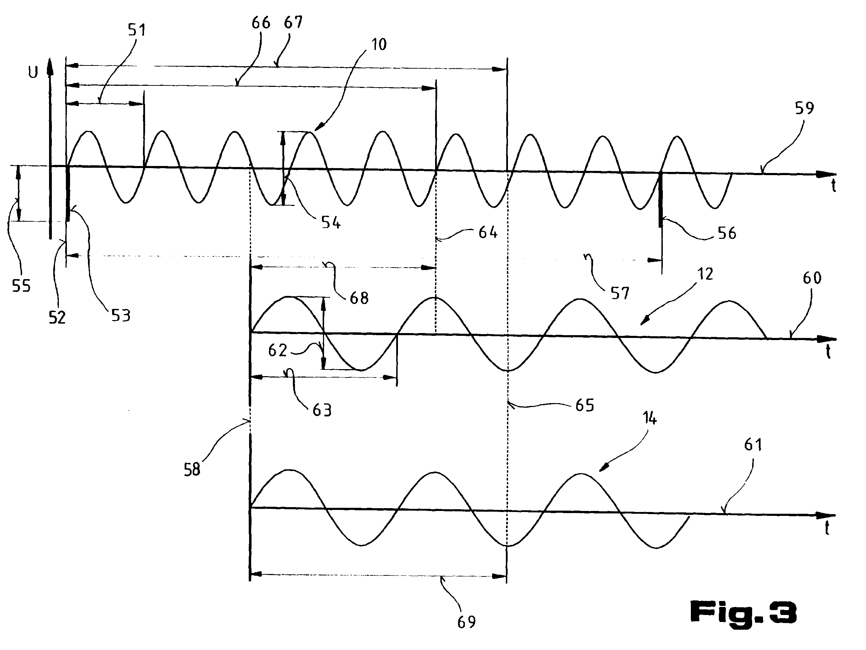

Anhand der Fig. 2 und 3 soll nun eine mögliche Ausführungsvariante der Positionsmeßeinrichtung 1 sowie der zur Ermittlung der Zeitdifferenz für ausgesendete bzw. empfangene Energiesendestrahlung 10 und Energierücksendestrahlung 12 bzw. 14 gezeigt und erläutert werden.

Wie bereits anhand der Übersichtsdarstellung in Fig. 1 gezeigt, sind am Rand einer Fläche 23, die im vorliegenden Fall durch einen Lagerplatz 40 gebildet ist, wiederum zwei stationär bzw. feststehend auf einem Basisstandort 6 und einem Nebenstandort 7 angeordnete Vermessungsvorrichtungen 3 und 4, die eine vorbestimmte Distanz 8 aufweisen, angeordnet.

Die Ausbildung der Vermessungsvorrichtungen 3 und 4 entspricht denjenigen, wie sie in Fig. 1 gezeigt sind.

Die Positionsmeßeinrichtung 1 besteht aus den Vermessungsvorrichtungen 3 und 4 dient nun dazu, die Manipulationswege beispielsweise zwischen Abstellplätzen 41 und 42 entsprechend zu ordnen oder entladene Waren 43, 44 an bestimmten Stellen des Lagerplatzes 40 geordnet abzustellen.

Dazu ist beispielsweise ein Hubstapler 45 angeordnet, der in verschiedenen Positionen 46, beispielsweise während einer Fortbewegung von der Position 46 bis 49, gezeigt ist.

Um nun die Position 46 bis 49 laufend überwachen zu können, beispielsweise um eine Fernsteuerung des Hubstaplers 45 zu ermöglichen oder eine optimale Fahrtroute auf dem Lagerplatz 40 zu kalkulieren bzw. den Energieaufwand und die Fahrweise des Bedieners zu kontrollieren, ist es nun erwünscht, entweder die Position 46 bis 49 oder die Geschwindigkeit bzw. die Beschleunigungen und die Stehzeiten genau zu erfassen bzw. über die Positionsmessung festzustellen, ob der Bediener mit dem Hubstapler 45 die Waren 43, 44 an der richtigen Stelle absetzt.

Dazu wird mit dem Sender 9 eine Energiesendestrahlung 10 mit einer Frequenz 51 zum Zeitpunkt 52 mit einem Startimpuls 53 bzw. Startsignal ausgesandt, welche durch elektromagnetische Wellen mit der Frequenz 51 z.B. zwischen 10 bis 100 MHz, bevorzugt 40 bis 60 MHz und mit einem sinusförmigen Verlauf und einer Amplitude 54 gebildet ist.

Die Energiesendestrahlung 10 sendet nun beispielsweise am Beginn eines Meßzyklus, also zu einem Zeitpunkt 52, einen Startimpuls 53 bzw. ein Startsignal, der eine Amplitude 55 aufweist, die sich charakteristisch von der Amplitude 54 der Energiesendestrahlung 10 unterscheidet. Dieser Startimpuls 53 bzw. dieses Startsignal kann natürlich auch aus mehreren Schwingungen der Energiesendestrahlung 10, also eines Hochfrequenz-Trägersignals bestehen. Die Zusammensetzung dieses Startimpulses 53 bzw. Startsignals ist aber in jedem Fall einzigartig, d.h. daß in den nachfolgend übermittelten Daten diese Signalform bzw. die Aufeinanderfolge dieser Signale nicht mehr vorkommt. Dieser Startimpuls 53 bzw. dieses Startsignal kennzeichnet den Beginn eines Meßzyklus. Die zwischen zwei solchen aufeinanderfolgenden Startimpulsen 53 und 56 bzw. Startsignale festgelegte Zeitspanne 57 legt die maximale Zeitdauer für einen Meßzyklus fest, in welchen neben einem Startimpuls 53 bzw. Startsignal gegebenenfalls auch der Identifikationscode 30 sowie sonstige Detailinformationen und die Laufzeitermittlung der Energiesendestrahlung 10 zwischen der Vermessungsvorrichtung 3 bzw. 4 und dem Hubstapler 45 erfolgt. Hat nun die Energiesendestrahlung 10 bezogen auf den Startimpuls 53 bzw. das Startsignal eine Entfernung la zwischen der Vermessungsvorrichtung 3 und dem Hubstapler 45 zurückgelegt, so wird beim Eintreffen des Startimpulses 53 bzw. Startsignales beim Positionsmelder 5, wie bereits anhand der Fig. 1 grob beschrieben, am Zeitpunkt 58 eine Energierücksendestrahlung 12 ausgestrahlt.

Die vom Positionsmelder 5 abgesandte Energierücksendestrahlung 12 bzw. 14 ist in dem in Fig. 3 dargestellten Diagramm, in welchem auf der Ordinate der Spannung U und auf der Abszisse die Zeit t eingetragen ist, gezeigt. Die Darstellung der elektromagnetischen Wellen, die die Energiesendestrahlung 10 und die Energierücksendestrahlung 12 bzw. 14 zeigen, erfolgt dabei schematisch und teilweise zur besseren Verständlichkeit grüßenmäßig verzerrt.

In diesem Diagramm ist nunmehr auf einer Grundlinie 59 die Energiesendestrahlung 10 und auf einer Grundlinie 60 und 61 die Energierücksendestrahlung 12 bzw. 14 auf der Grundlinie 60 bezogen auf die Vermessungsvorrichtung 3 und auf der Grundlinie 61 bezogen auf die Vermessungsvorrichtung 4 dargestellt. Es wird der Einfachheit halber nunmehr davon ausgegangen, daß nach Einlangen des Startimpulses 53 bzw. Startsignales beim Positionsmelder 5 dieser über seinen Sender 16 die Energierücksendestrahlung 12 aussendet. Diese kann beispielsweise eine annähernd gleiche Amplitude 62, wie die Amplitude 54 der Energiesendestrahlung 10 aufweisen. Die Energierücksendestrahlung 12 weist gegenüber der Frequenz 51 der Energiesendestrahlung 10 eine unterschiedliche, im vorliegenden Fall die halbe Frequenz 63 auf. Entsprechend der Entfernung la zwischen dem Hubstapler 45 und der Vermessungsvorrichtung 3, die kleiner ist als eine Entfernung lb zwischen dem Hubstapler 45 und der Vermessungsvorrichtung 4, sodaß die Energierücksendestrahlung 12 früher beim Empfänger 11 der Vermessungsvorrichtung 3 eintrifft als die Energierücksendestrahlung 14 am Empfänger 13 der Vermessungsvorrichtung 4, wie dies auch anhand des dargestellten Signal-verlaufes auf den Grundlinien 60, 61 zu ersehen ist. Somit trifft die Energierücksendestrahlung 12 bzw. 14 zum Zeitpunkt 64 nach Zurücklegen der Entfernung 1a bei der Vermessungsvorrichtung 3 und zu einem Zeitpunkt 65 nach Zurücklegen der Entfernung lb bei der Vermessungsvorrichtung 4 ein. Unter Berücksichtigung einer feststehenden Distanz lc zwischen den beiden Vermessungsvorrichtungen 3 und 4 läßt sich die Position des Positionsmelders 5 daraus ableiten.

Wird nach Einlangen der Energierücksendestrahlung 12 bzw. 14 an der jeweiligen Vermessungsvorrichtung 3 und 4 die Zählereinheit 34 stillgesetzt bzw. der Zählerstand abgefragt, so können dadurch zwei Zeitsignale mit einer Zeitdauer 66 und einer Zeitdauer 67 ermittelt werden, die der Entfernung la und lb proportional sind.

In der gemessenen Zeitdauer 66 ist eine Laufzeit der Energiesendestrahlung 10 von der Vermessungsvorrichtung 3 zum Positionsmelder 5 enthalten und beinhaltet intern eine Laufzeit der Energierücksendestrahlung 12 und eine Laufzeit der Elektronik im Positionsmelder 5. Die Laufzeit der Elektronik kann man messen oder durch geeignete schaltungstechnische Maßnahmen wie Synchronisation zu Null machen. Infolge läßt sich aus summierten Gesamtlaufzeiten die Laufzeit für die Entfernungen la ermitteln. Ebenso verhält es sich für die Entfernung lb.

Die Position 46 des Hubstaplers 45 auf dem Lagerplatz 40 errechnet sich nun wie folgt.

$$la^2 = lb^2 + lc^2 - 2 * lb * lc * \cos\alpha \qquad => \qquad \alpha = \arccos\left(\frac{la^2 - lb^2 - lc^2}{-2 * lb * lc}\right)$$

$$lb^2 = la^2 + lc^2 - 2 * la * lc * \cos\beta \qquad => \qquad \beta = \arccos\left(\frac{lb^2 - la^2 - lc^2}{-2 * la * lc}\right)$$

$$lc^2 = la^2 + lb^2 - 2 * la * lb * \cos\gamma \qquad => \qquad \gamma = \arccos\left(\frac{lc^2 - la^2 - lb^2}{-2 * la * lb}\right)$$

$$\cos\beta = \left(\frac{lc_1}{la}\right) \qquad => \qquad lc_1 = la \cos\beta$$

$$\cos\alpha = \left(\frac{lc_2}{lb}\right) \qquad => \qquad lc_2 = lb \cos\alpha$$

$$lb^2 = lc_2^2 + h^2 => h = \sqrt{(lb^2 - lc_2^2)}$$

$$la^2 = lc_1^2 + h^2 => h = \sqrt{(la^2 - lc_1^2)}$$

Daraus ergeben sich nunmehr aufgrund der festgestellten Zeitdauer von ta und tb die Entfernung 31 und 32 sowie die Position 46 bis 49 des Objektes 2 in einem Koordinatensystem unter Berücksichtigung, daß sich die Energiesendestrahlung 10 und die Energierücksendestrahlung 12,14 mit Lichtgeschwindigkeit = $300 * 10^6$n/s ausbreitet.

Zur weiteren Erläuterung des Berechnungsbeispieles, anhand der in Fig. 2 gezeigten verschiedenen Positionen 46 bis 49 des Hubstaplers 45, wurden nunmehr folgende Werte aus den für die Entfernungen 31 und 32 ermittelten Laufzeiten, anhand der zuvor erläuterten Berechnungsmethoden für die Errechnung der tatsächlichen Zeitdauer 68 und 69 eines Referenzsignals mit einer vorgegebenen Frequenz mit den Werten ta und tb errechnet:

| | |
|---|---|
| t1a = 232 ns | t1b = 175 ns |
| t2a = 234 ns | t2b = 174 ns |
| t3a = 235 ns | t3b = 173 ns |
| t4a = 236 ns | t4b = 173 ns |

Die Entfernung lc ist eine feste Größe. Sie kann auf verschiedene Arten ermittelt werden, wie z.B. durch Abmessen der Entfernung lc mit einem Maßband oder ebenfalls durch Messen der Laufzeit von der Vermessungsvorrichtung 3 bis zur Vermessungsvorrichtung 4. Ist die Laufzeit der Entfernung lc bekannt, errechnet sich der Abstand wie folgt

$$lc = tc * f * \lambda = tc * c = 300 \cdot 10^{-9} * 300 \cdot 10^6 = 90 \text{ m}$$

Strecke la:

ta = 232 ns

$$la = ta * f * \lambda = ta * c = 232 \; 10^{-9} * 300 \; 10^{6} = 69,6 \; m$$

Strecke lb:

tb = 175 ns

$$lb = tb * f * \lambda = tb * c = 175 \; 10^{-9} * 300 \; 10^{6} = 52,5 \; m$$

Winkel $\alpha$:

$$\alpha = \arccos\left(\frac{la^2 - lb^2 - lc^2}{-2 * lb * lc}\right) = \arccos\left(\frac{69,6^2 - 52,5^2 - 90^2}{-2 * 52,5 * 90}\right) = 50,49°$$

Winkel $\beta$:

$$\beta = \arccos\left(\frac{lb^2 - la^2 - lc^2}{-2 * la * lc}\right) = \arccos\left(\frac{52,5^2 - 69,6^2 - 90^2}{-2 * 69,6 * 90}\right) = 35,59°$$

Strecke $lc_1$:

$$lc_1 = la \cos \beta = 69,6 \cos 35,59° = 56,6 \; m$$

Strecke $lc_2$:

$$lc_2 = lb \cos \alpha = 52,5 \cos 50,49° = 33,4 \; m$$

Strecke h:

$$h = \sqrt{(lb^2 - lc_2^2)} = \sqrt{(la^2 - lc_1^2)} = \sqrt{(52,5^2 - 33,4^2)} = \sqrt{(69,6^2 - 56,6^2)} = 40,5\,m$$

Mit diesen Werten ist nun die Position 46 des bewegten Objektes 2 relativ zur Vermessungsvorrichtung 3 und der Vermessungsvorrichtung 4 bestimmt. Zwar würde ein Objekt 2, das an einer Verbindungslinie zwischen Vermessungsvorrichtung 3 und 4 gespiegelt wird, dieselben Meßergebnisse aufweisen, aber durch eine geeignete Aufstellung von den Vermessungsvorrichtungen 3 und 4 und eine Bereichsvereinbarung z.B. des Lagerplatzes 40 kann dieser Bereich ausgeschlossen werden.

Die Ermittlung der entsprechenden Positionswerte für die weiteren Positionen 47 bis 49 des Hubstaplers 45 erfolgt in gleicher Weise.

Sind diese Werte dann berechnet, kann der jeweils zurückgelegte Weg des Hubstaplers 45, beispielsweise zwischen den einzelnen Positionen 46 bis 49 sowie die dazwischen erzielte Geschwindigkeit und die Beschleunigung wie folgt errechnet werden:

$$lc_1 = la\cos\beta = ta * c * \left( \frac{(tb*c)^2 - (ta*c)^2 - (tc*c)^2}{(-2*ta*tc*c^2)} \right)$$

$$h = \sqrt{\left(la^2 - lc_1^2\right)} = \sqrt{(ta*c)^2 - \left( ta * c * \left( \frac{(tb*c)^2 - (ta*c)^2 - (tc*c)^2}{(-2*ta*tc*c^2)} \right) \right)^2}$$

Aus der Positionsberechnung können folgende relative Abstände errechnet werden:

$$11c_1 = 56,599 \text{ m} \qquad h1 = 40,505 \text{ m}$$
$$12c_1 = 57,240 \text{ m} \qquad h2 = 40,640 \text{ m}$$
$$13c_1 = 57,648 \text{ m} \qquad h3 = 40,582 \text{ m}$$
$$14c_1 = 57,883 \text{ m} \qquad h4 = 40,769 \text{ m}$$

Teilstrecke s:

$$s = \sqrt{\Delta x^2 + \Delta y^2)}$$

Aus den errechneten Positionen ergeben sich somit folgende Teilstrecken:

$$\Delta x12 = 0,640 \text{ m} \qquad Dy12 = 0,135 \text{ m} \quad S12 = 0,655 \text{ m}$$
$$\Delta x23 = 0,408 \text{ m} \qquad Dy23 = 0,057 \text{ m} \quad S23 = 0,412 \text{ m}$$
$$\Delta x34 = 0,235 \text{ m} \qquad Dy34 = 0,187 \text{ m} \quad S34 = 0,300 \text{ m}$$

Daraus folgt eine Gesamtstrecke S8;

$$S_8 = \Sigma S = S12 + S23 + S34 = 0,655 + 0,412 + 0,300 = 1,367 \text{ m}$$

Die Berechnung der Geschwindigkeit erfolgt derart, daß zuerst die Geschwindigkeit des Objektes 2 auf einer Teilstrecke ermittelt wird.

Ermittelte Teilstrecken:

S12 = 0,655 m
S23 = 0,412 m
S34 = 0,300 m

Meßintervall:

tm = 0,1 s

Diese Zeitdauer von 100 ms zwischen den einzelnen Messungen dient nur als Beispiel. Die tatsächliche Meßintervallzeit der Positionsmeßeinrichtung 1 kann viel geringer sein und im $\mu$s-Bereich liegen.

Geschwindigkeit v:

$$v = \frac{s}{tm}$$

Auf den ermittelten Teilstrecken S12-S34 werden folgende Geschwindigkeiten des Hubstaplers 45 erreicht.

V12 = 6,55 m/s = 23,58 km/h
V23 = 4,12 m/s = 14,83 km/h
V34 = 3,00 m/s = 10,80 km/h

Der große Geschwindigkeitsunterschied kommt vom großen Meßintervall. Bei einem kürzeren Meßintervall erhält man kleinere Positionsänderungen und somit werden die Sprünge im Meßergebnis immer kleiner.
Die Beschleunigung kann anhand der vorliegenden Daten durch die Berechnung der Beschleunigung des Hubstaplers 45 auf einer Teilstrecke ermittelt werden, wobei von den ermittleren Geschwindigkeiten für die Teilstrecke S12-S34 ausgegangen wird:

V12 = 6,55 m/s = 23,58 km/h
V23 = 4,12 m/s = 14,83 km/h
V34 = 3,00 m/s = 10,80 km/h

Aus diesen Geschwindigkeiten errechnet sich nun die Beschleunigung a wie folgt:

$$a = \frac{\Delta v}{\Delta t} = \frac{(vt+1-vt)}{tm}$$

Auf den ermittelten Teilstrecken S12-S34 liegen danach folgende Beschleunigungen des Hubstaplers 45 vor:

a12-23 = 24,3 m/s$^2$
a23-34 = 11,2 m/s$^2$

Für den großen Beschleunigungsunterschied gilt dasselbe wie für den Geschwindigkeitsunterschied. Je kürzer das Meßintervall, desto mehr nähert man sich einer Echtzeitdarstellung an. Mit einer derartigen Positionsmeßeinrichtung 1 ist es daher möglich, sowohl Echtzeitdarstellungen und Aufnahmen wiederzugeben, als auch für die Archivierung nachfolgende Auswertung zeitversetzter Aufzeichnungen vorzunehmen.

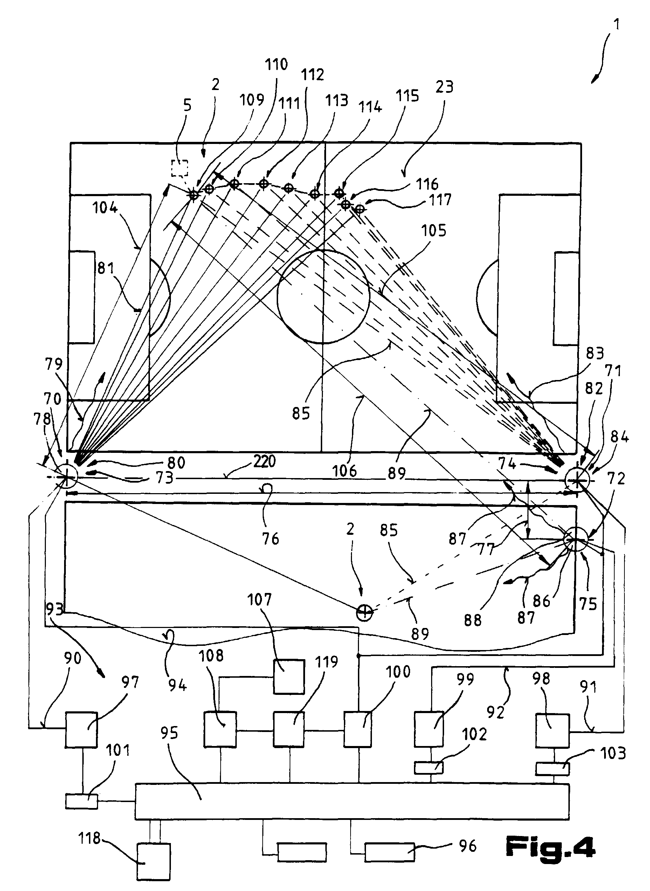

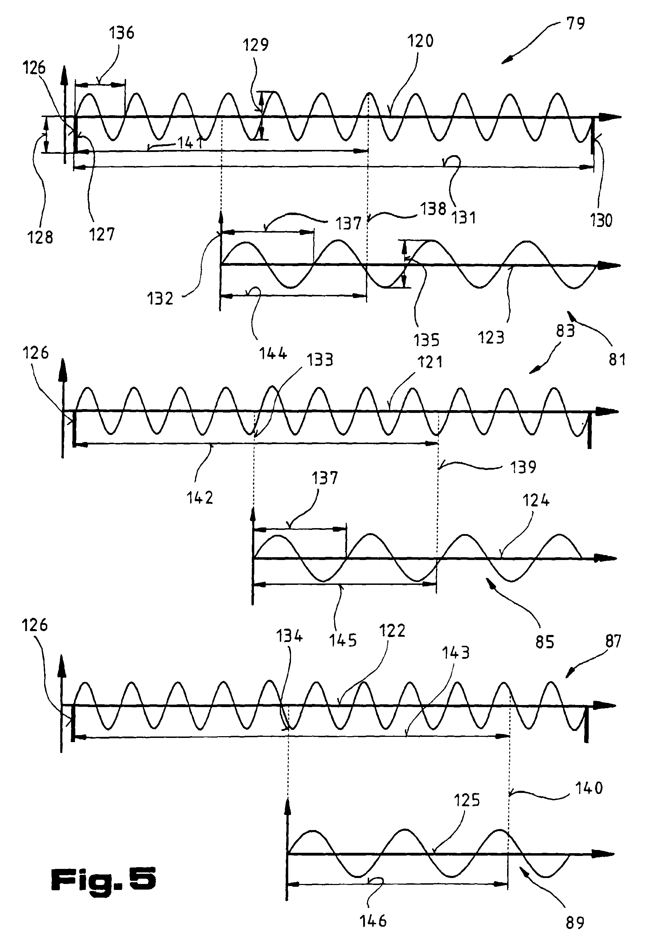

In den Fig. 4 und 5 sollen nun eine andere mögliche Ausführungsvariante der Positionsmeßeinrichtung 1 zur Bestimmung der Position des Objektes 2 erläutert werden.

Die Positionsmeßeinrichtung 1 besteht dabei aus einer ersten Vermessungsvorrichtung 70, einer zweiten Vermessungsvorrichtung 71 und einer dritten Vermessungsvorrichtung 72 und dem an dem Objekt 2 angeordneten Positionsmelder 5.

Der Positionsmelder 5 ist gemeinsam mit dem Objekt 2 beweglich angeordnet. wogegen die Vermessungsvorrichtungen 70, 71 und 72 stationär auf einem Basisstandort 73, einem Nebenstandort 74 und einem weiteren Nebenstandort 75 angeordnet sind. Die Vermessungsvorrichtung 70, 71 sind bevorzugt während des Meßvorganges bzw. einer gesamten Auswertung in einer fixen Distanz 76 und die Vermessungsvorrichtung 71, 72 in einem Abstand 77 voneinander angeordnet. Selbstverständlich ist es möglich diese Distanzen 76 und den Abstand 77 jeweils vor Beginn einer Meßserie neu festzulegen, es ist jedoch auch möglich, durch ständige Überwachung dieser Distanz zueinander, auch die Vermessungsvorrichtungen 70 bis 72 relativ zueinander zu bewegen. Im letztgenannten Fall sind nur jeweils vor der Berechnung der einzelnen Entfernungen der Positionsmelder von den Vermessungsvorrichtungen 70 bis 72 die Distanzen 76 und den Abstand 77 zwischen den Vermessungsvorrichtungen 70 bis 72 zu ermitteln.

Die am Basisstandort 73 angeordnet Vermessungsvorrichtung 70 umfaßt einen Sender 78 zum Aussenden einer Energiesendestrahlung 79 und einen Empfänger 80 zum Empfangen einer Energierücksendestrahlung 81. Die am ersten Nebenstandort 74 angeordnete Vermessungsvorrichtung 71 umfaßt wiederum einen Sender 82 zum Aussenden einer Energiesendestrahlung 83 und einen Empfänger 84 zum Empfangen einer Energierücksendestrahlung 85. Weiters umfaßt die dritte Vermessungsvorrichtung 72 am Nebenstandort 75 einen Sender 86 zum Aussenden einer Energiesendestrahlung 87 und einen Empfänger 88 zum Empfangen einer Energierücksendestrahlung 89. Der Positionsmelder 5 ist, wie bereits anhand der Übersichtsdarstellung in Fig. 1 gezeigt. ausgeführt.

Die Vermessungsvorrichtungen 70, 71 und 72 sind über Leitungen 90 bis 92 mit einer Steuereinrichtung 93 verbunden. Für die Ermittlung und/oder Überwachung und gegebenenfalls der Auswertung der Daten von der Position eines Objektes 2 innerhalb der Fläche 23 oder einer Fläche 94, also einer Spielfläche, ist die Steuereinrichtung 93, die durch eine Rechnereinheit 95 gebildet sein kann, dargestellt. Die Rechnereinheit 95 wird über ein Eingabegerät 96 angesteuert. Dabei werden die von der Rechnereinheit 95 die von einem Eingabegerät 96 angegebenen Daten auf einem Ausgabegerät, der z.B. aus einer Displayanzeige oder einem Bildschirm gebildet ist, angezeigt.

Zur Herstellung der Energiesendestrahlungen 79, 83 und 87 und der Auswertung der Energierücksendestrahlungen 81, 85 und 89 ist die Rechnereinheit 95 mit drei Sendemodulen 97 bis 99 und einem Empfängermodul 100 ausgestattet.

Weiters weist die Rechnereinheit 95 für jedes Sendemodul 97 bis 99 ein Codiermodul 101 bis 103 auf, mit dem jeder Energiesendestrahlung 79, 83 und 87 ein entsprechender Identifikationscode 30 überlagert werden kann. Dieser Identifikationscode 30 ermöglicht es, jeweils einzelne Positionsmelder 5 anzusprechen, sodaß dieser zur Feststellung ihrer Entfernung 104 bis 106 von den Vermessungsvorrichtungen 70 bis 72 bzw. dem Basisstandort 73 und den Nebenstandorten 74, 75 die Energierücksendestrahlungen 81, 85 und 89 an diesen Vermessungsvorrichtungen 70 bis 72 abgesetzt werden kann. Die Entfernungen 104 bis 106 werden über das Zeitäquivalent der Laufzeit der Energiesendestrahlung 79, 83 und 87 und der Energierücksendestrahlungen 81, 85 und 89 ermittelt.

Weiters können die Energierücksendestrahlungen 81, 85 und 89 ebenfalls den Identifikationscode 30 aufweisen, dies muß aber nicht unbedingt mitgesendet werden.

Zur Laufzeiterfassung ist in der Rechnereinheit 95 ein Taktgenerator 107, eine Zählereinheit 108 und eine entsprechende Ausbildung bzw. Softwareausstattung in der Rechnereinheit 95 vorgesehen. Zum Feststellen der Position jenes Objektes 2 kann beispielsweise über eine Befehlseingabe in dem Eingabegerät 96 z.B. eine Tastatur eine Bezeichnung des Objektes 2 eingegeben werden, dessen Objektgeschwindigkeit oder - beschleunigung ermittelt werden soll. In unserem Ausführungsbeispiel soll nun eine Wegmessung eines Objektes 2 von einer Position 109 über die Positionen 110 bis 116 bis zu einer Position 117 ermittelt werden.

Über die Festlegung des Objektes 2 wird aus einem Speicher 118 der entsprechende Identifikationscode 30 abgefragt und über die Codiermodule 101 bis 103 dieser Identifiaktionscode 30 über die Sendemodule 97 bis 99 den jeweiligen Sendern 78, 82 und 86 in der Vermessungsvorrichtung 70, 71 und 72 zugeführt und von diesem ausgesandt.

Gleichzeitig mit der Aktivierung der einzelnen Sendemodule 97 bis 99 wird die Zählereinheit 108 synchron gestartet und zählt die von dem Taktgenerator 107 gelieferten Zeitimpulse. Langt nun die ausgestrahlte Energiesendestrahlung 79 im Empfänger 15 des Positionsmelders 5 ein, so wird über die Decodiereinheit 17 verglichen, ob der in der Energiesendestrahlung 79 enthaltene Identifikationscode 30 mit dem in einem Speicher 39 des Positionsmelder 5 hinterlegten Identifikationscode 30 übereinstimmt. Ist eine Übereinstimmung gegeben, so wird die Absendung der Energierücksendestrahlung 81 mit dem jeweiligen Identifikationscode 30 ausgelöst. Dieser Vorgang wiederholt sich für jede der von den Vermessungsvorrichtungen 71 und 72 ausgesandten Energiesendestrahlungen 83 und 87 und strahlt nach Vergleich des Identifikationscodes 30 diesen wieder über die Energierücksendestrahlungen 85 und 89 zurück.

Langt nunmehr die Energierücksendestrahlung 81 und gegebenenfalls 85 und 89 am Empfänger 80, 84 und 88 der Vermessungsvorrichtung 70 bis 72 ein, so wird über das Empfängermodul 100 der Identifkationscode 30 herausgefiltert und einem Decodiermodul 119 übergeben. Gleichzeitig mit dem Übergeben des Identifkationscodes 30 an das Decodiermodul 119 wird von dem Empfängermodul 100 ein Steuersignal an die Zählereinheit 108 abgegeben, welches beispielsweise bewirkt, daß der jeweilige Zählerstand in dem Speicher 118 der Rechnereinheit 95 hinterlegt wird. Anschließend weist die Rechnereinheit 95 den decodierten Identifikationscode 30 zum Zählerstand im Speicher 118 zu.

Aufgrund der zwischen dem Start- und dem Stoppimpuls ermittelten Anzahl der vom Taktgenerator 107 abgegebenen Taktimpulse wird die Laufzeit, die die Energiesendestrahlung 79, 83, 87 vom Sender 78, 82, 86 bis zum Positionsmelder 5 und von diesen die Energierücksendestrahlung 81, 85, 89 zurück zu den Empfängern 80, 84, 88 der Vermessungsvorrichtung 70 bis 72 benötigt hat, erfaßt.

Anhand der in Fig. 5 dargestellten Energiesendestrahlungen 79, 83 und 87 und der Energierücksendestrahlungen 81, 85, 89 wird nun vom Aussenden der Energiesendestrahlung 79, 83 und 87 von den Sendern 78, 82, 86 der Vermessungsvorrichtung 70, 71 und 72 bis zum Empfangen der Energierücksendestrahlungen 81, 85, 89 auf den Empfänger 80, 84 und 88 der Vermessungsvorrichtungen 70, 71 und 72 die Laufzeitermittlung näher erläutert.

Die von den Vermessungsvorrichtungen 70, 71 und 72 ausgesendeten Energiesendestrahlungen 79, 83 und 87 und die vom Positionsmelder 5 abgesandten Energierücksendestrahlungen 81, 85 und 89 sind in den in Fig. 5 gezeigten Diagrammen, in welchen auf der Ordinate die Spannung V und auf der Abszisse die Zeit t eingetragen ist in Form eines Sinussignals dargestellt. Die Darstellung der elektromagnetischen Wellen, die die Energiesendestrahlung 79, 83 und 87 und die Energierücksendestrahlungen 81, 85 und 89 zeigen, erfolgt dabei schematisch und teilweise zur besseren Verständlichkeit größenmäßig verzerrt, und es kann ohnedies anstelle der Sinusform jede andere Signalform verwendet werden.

In diesen Diagrammen sind nunmehr auf einer Grundlinie 120, 121 und 122 die Energiesendestrahlungen 79, 83 und 87 und auf einer Grundlinie 123, 124, 125 die Energierücksendestrahlungen 81, 85 und 89 dargestellt.

Die Energiesendestrahlungen 79, 83, 87 senden nun beispielsweise am Beginn eines Meßzyklus, also zu einem Zeitpunkt 126, beispielsweise wieder einen Startimpuls 127 bzw. ein Startsignal aus der eine Amplitude 128 aufweist, die sich charakteristisch von einer Amplitude 129 der Energiesendestrahlungen 79, 83 und 87 unterscheidet. Dieser Startimpuls 127 bzw. ein zuvor erwähntes Zeitsignal kennzeichnet den Beginn eines Meßzyklus, welcher durch zwei solche aufeinanderfolgende Startimpulse 127 und 130 bzw. Startsignale festgelegt ist, wobei die Zeitspanne 131 die maximale Dauer für einen Meßzyklus festlegt, in welchem neben einem Startimpuls 127 bzw. Startsignal gegebenenfalls auch der Identifikationscode 30 sowie sonstige Detailinformationen übermittelt werden. Gleichzeitig wird durch die Zeitspanne 131, also die maximale Dauer des Meßzyklus, die maximale Entfernung begrenzt, die ein Objekt 2 von einem Basisstandort 73 und/oder Nebenstandorten 74,75 haben kann. Selbstverständlich ist es auch möglich, die einzelnen Abfragen der Positionen 109 bis 117 von verschiedenen Objekten 2 einander zu überlagern, indem die maximal mögliche Entfernung eines Objekts 2 von der Positionsmeßeinrichtung 1 bzw. deren Vermessungsvorrichtungen 70 bis 72 festgelegt wird, die minimale Zeitdauer, innerhalb welcher die Startimpulse bzw. Startsignale für die Feststellung der Position von gleichen oder verschiedenen Objekten 2 ausgelöst werden kann, setzt sich aus der Dauer des Identifikationscodes 30 der einzelnen Objekte 2 zuzüglich einer oder mehrerer eventueller Startimpulse 127 bzw. Startsignale und sonstiger für die wichtige Zuordnung der Sende- und Empfangssignale notwendiger Daten, sowie gegebenenfalls bicharakteristischen Merkmale der jeweiligen Senderstrahlung aufcodierten Information zusammen. Z. B. ist es möglich, mit dem Identifikationscode 30 gleichzeitig jenen Zählerstand in codierter Form mitzusenden. der bei der Absendung des Startimpulses bzw. Startsignals von der Vermessungsvorrichtung 70 bis 72 vorlag.

Je dichter die Energiesendestrahlung 79, 83 und 87 bzw. die auf diese aufmodulierten Information, wie beispielsweise Startimpulse 127 bzw. Startsignale und Identifikationscode 30 gesandt wird, umso größer ist das Risiko einer Differenz zwischen diesen Signalen. sodaß gegebenenfalls bei gleichzeitig einlangenden Signalen gleicher Frequenz der eine oder andere Berechnungsvorgang unterdrückt werden muß, oder es wird mit unterschiedlichen Frequenzen gearbeitet bzw. die Zeitspanne zwischen der Absendung der verschiedenen Identifikationscodes vergrößert. Ist die Zeitspanne 131 so gewählt, daß sie ausreicht, daß die Energiesendestrahlung 79,83 und 87 das weitest mögliche Objekt 2 erreicht und die von diesen ausgesendete Energierücksendestrahlung 81, 85 und 89 wieder in der Vermessungsvorrichtung 70 bis 72 eingelangt ist, so können keine Signalüberlagerungen und dergleichen auftreten. Hat nun die Energiesendestrahlung 79 bezogen auf den Startimpuls 127 bzw. das Startsignal eine Entfernung 104 zwischen der Vermessungsvorrichtung 70 und dem Objekt 2 zurückgelegt, so wird beim Eintreffen des Startimpulses 127 bzw. Startsignales beim Positionsmelder 5, wie bereits anhand der Fig. 1 grob beschrieben, eine Energierücksendestrahlung 81 mit dem Identifikationscode 30 ausgestrahlt.

In gleicher Weise wie nunmehr für die Aussendung der Energiesendestrahlung 79 und der Energierücksendestrahlung 81 beschrieben, wird nun beim Einlangen der Startimpulse 127 bzw. Startsignale beim Positionsmelder 5 über dessen Sender 16 die Energierücksendestrahlung 85 und 89 abgesandt. Daraus ist zu ersehen, daß der Sendebeginn der Energierücksendestrahlungen 81, 85 und 89 bei der vorliegenden Position des Objektes 2 zu verschiedenen Zeitpunkten 132, 133 und 134 stattfindet. Entsprechend der Entfernung 104 zwischen dem Objekt 2 und der Vermessungsvorrichtung 70, die kleiner ist als eine Entfernung 105 zwischen dem Objekt 2 und der Vermessungsvorrichtung 71 und die wiederum kleiner ist als eine Entfernung 106 zwischen dem Objekt 2 und der Vermessungsvorrichtung 72, trifft die Energierücksendestrahlung 81 als erste bei einem Empfängermodul 100 der Rechnereinheit 95 ein und somit vor der Energierücksendestrahlung 85 und der Energierücksendestrahlung 89. Lediglich der guten Ordnung halber sei an dieser Stelle erwähnt, daß selbstverständlich die Energiesende und die Energierücksendestrahlung 79, 83, 87 und 81, 85, 89 gleiche oder unterschiedliche Amplituden 135, bevorzugt jedoch unterschiedliche Frequenzen 136 und 137 aufweisen. Die Energierücksendestrahlung 81 trifft also zum Zeitpunkt 138, die Energierücksendestrahlung 85 zum Zeitpunkt 139 und die Energierücksendestrahlung 89 zum Zeitpunkt 140 am Empfängermodul 100 der Rechnereinheit 95 ein.

Wird nach Einlangen der Energierücksendestrahlungen 81, 85 und 89 an der jeweiligen Vermessungsvorrichtung 70 bis 72 der Zählerstand an die Zählereinheit 108 abgefragt, so können dadurch drei Zeitsignale mit einer Zeitdauer 141, einer Zeitdauer 142 und einer Zeitdauer 143 ermittelt werden, die den Entfernungen 104 bis 106 von den Vermessungsvorrichtungen 70 bis 71 bis zum Positionsmelder 5 und wieder zurück proportional sind.

Aus der gesamten Zeitdauer 141 bis 143 errechnet sich nunmehr die Zeitdauer 144 bis 146, also die Zeitdauer 144 bis 146, die die Energierücksendestrahlung 81, 85 und 89 zum Zurücklegen vom Objekt 2 zur jeweiligen Vermessungsvorrichtung 70 bis 71 mit der abgezogenen Laufzeit der Elektronik tatsächlich benötigen.

Mit dieser Vermessungsvorrichtung 72 ist in diesem Ausführungsbeispiel eine Bestimmung der Position von Objekten 2, die sich auf den Flächen 23 und 94 befinden, mit nur einer Positionsmeßeinrichtung 1 möglich. Die Feststellung, ob sich das Objekt 2 in der Fläche 23 oder in der Fläche 94 befindet, erfolgt über die zusätzlich angeordnete Vermessungsvorrichtung 72, indem die Zeitdauer 145 und 146, die die Energieücksendestrahlung 81, 85, 89 vom Objekt 2 zu den Vermessungsvorrichtungen 71, 72 benötigt, gegenübergestellt wird. Ist die Zeitdauer 145 der Vermessungsvorrichtung 71 kleiner als die Zeitdauer 146 der Vermessungsvorrichtung 72, so befindet sich das Objekt 2 auf der Fläche 23 oder bei langer Zeitdauer 145 auf der Fläche 94.

Die Berechnung der Positionen 109 bis 117 kann nun anhand der Formeln, wie sie anhand des Ausführungsbeispieles in Fig. 2 und 3 erläutert wurden, erfolgen. Die Berechnungen für die Zeitdauer 146 der Vermessungsvorrichtung 72 ist aus Übersichtsgründen nicht dargestellt, da sie gleich abläuft, wie bei den Vermessungsvorrichtungen 70, 71, und nur zum Vergleich zwischen der Zeitdauer 145 und der Zeitdauer 146 dient, um festzustellen, ob sich das Objekt 2 auf der Fläche 23 oder 94 befindet.

Zur weiteren Erläuterung des Berechnungsbeispieles anhand der in Fig. 4 gezeigten verschiedenen Positionen des Objektes 2 wurden nunmehr folgende Werte für die Zeitdauer 144 => ta und für die Zeitdauer 145 => tb gemessen.

Die Zeitdauer tc ergibt sich aus der Zeitmessung und damit der Distanz 76 zwischen den Vermessungsvorrichtungen 70 und 71.

| Position | ta/ns | tb/ns | tc/ns |
|---|---|---|---|
| 109 | 200.033 | 312.700 | 333.333 |
| 110 | 207.233 | 306.700 | 333.333 |
| 111 | 217.900 | 295.766 | 333.333 |
| 112 | 228.366 | 280.733 | 333.333 |
| 113 | 235.333 | 266.366 | 333.333 |
| 114 | 243.333 | 252.300 | 333.333 |
| 115 | 254.600 | 241.166 | 333.333 |
| 116 | 252.200 | 233.933 | 333.333 |
| 117 | 257.200 | 224.933 | 333.333 |

Aus den gemessenen Laufzeiten ergeben sich folgende Strecken:

| Position | la/m | lb/m | lc/m |
|---|---|---|---|
| 109 | 60.009 | 93.810 | 100.000 |
| 110 | 62.169 | 92.010 | 100.000 |
| 111 | 65.370 | 88.729 | 100.000 |
| 112 | 68.509 | 84.219 | 100.000 |
| 113 | 70.599 | 79.090 | 100.000 |
| 114 | 72.999 | 75.690 | 100.000 |
| 115 | 76.380 | 72.349 | 100.000 |
| 116 | 75.660 | 70.179 | 100.000 |
| 117 | 77.160 | 67.479 | 100.000 |

Daraus ergeben sich folgende Abstände relativ zu den Vermessungsvorrichtungen 70 und 71:

| Position | $lc_1$/m | h/m |
|---|---|---|
| 109 | 24.004 | 54.999 |
| 110 | 26.996 | 56.002 |
| 111 | 32.001 | 57.001 |
| 112 | 38.003 | 57.003 |
| 113 | 42.993 | 55.998 |
| 114 | 48.000 | 54.999 |
| 115 | 52.997 | 55.001 |
| 116 | 53.996 | 52.998 |
| 117 | 57.000 | 52.005 |

Mit diesen Werten ist nun die Position des bewegten Objektes 2 relativ zur Vermessungsvorrichtung 70 und Vermessungsvorrichtung 71 bestimmt. Zwar würde ein Objekt 2, das an der Verbindungslinie zwischen den Vermessungsvorrichtungen 70, 71 gespiegelt wird, dieselben Meßergebnisse aufweisen, aber durch den Vergleich der Zeitdauer 145 und 146 der Vermessungsvorrichtung 71 und 72 kann die Rechnereinheit 95 die Position des Objektes 2 den Flächen 23 oder 94, auf welchen sich das Objekt 2 befindet, zuordnen.

Aus diesen Werten ergibt die in den einzelnen Meßintervallen zurückgelegte Strecke:

| Position | s/m |
|---|---|
| 109-110 | 3.155 |
| 110-111 | 5.103 |
| 111-112 | 6.001 |
| 112-113 | 5.090 |
| 113-114 | 5.104 |
| 114-115 | 4.997 |

(fortgesetzt)

| Position | s/m |
|---|---|
| 115-116 | 2.238 |
| 116-117 | 3.164 |
| 109-117 | 34.852 |

Die Objektgeschwindigkeit auf den Teilstrecken beträgt:

| Position | v/m/s | v/km/h |
|---|---|---|
| 109-110 | 3.155 | 11.358 |
| 110-111 | 5.103 | 18.370 |
| 111-112 | 6.001 | 21.603 |
| 112-113 | 5.090 | 18.324 |
| 113-114 | 5.104 | 18.374 |
| 114-115 | 4.997 | 17.989 |
| 115-116 | 2.238 | 8.056 |
| 116-117 | 3.164 | 11.390 |
| 109-117 | 4.356 | 15.681 |

Die Objektbeschleunigung auf den Teilstrecken beträgt somit:

| Position | $v/m/s^2$ |
|---|---|
| 12-23 | 7.012 |
| 23-34 | 3.233 |
| 34-45 | -3.279 |
| 45-56 | 0.050 |
| 56-67 | -0.385 |
| 67-78 | -9.933 |
| 78-89 | 3.334 |

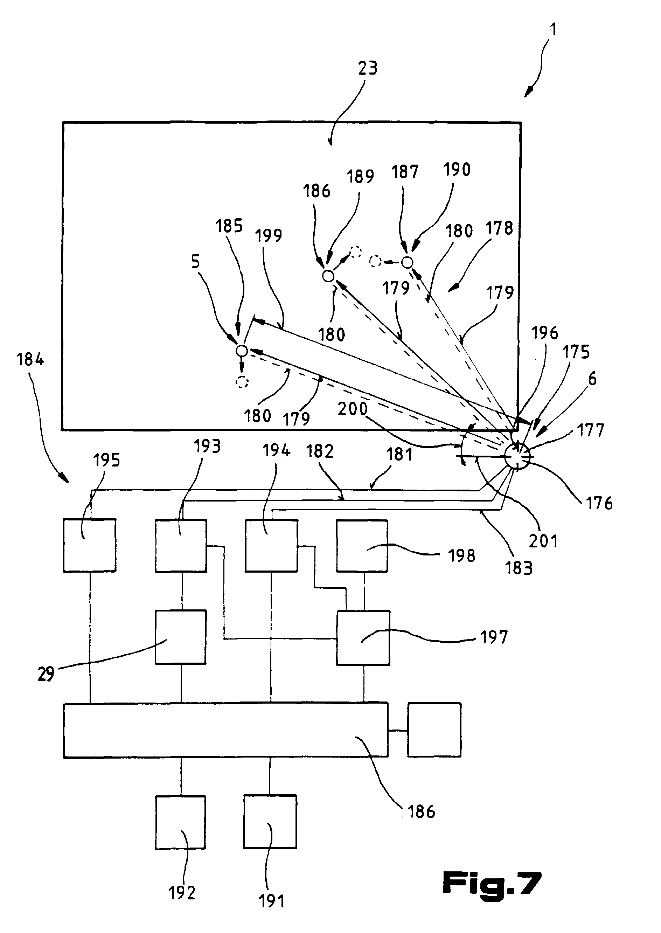

Anhand der Fig. 6 soll nun eine mögliche Ausführungsvariante der Positionsmeßeinrichtung 1 zur Ermittlung der Geschwindigkeit eines auf einer Fläche 23 befindlichen Objektes 2, z.B. eines 100-m-Läufers, erläutert werden.

Wie bereits anhand der in Fig. 4 gezeigten Ausführungsvariante sind am Rand einer Fläche 23, die im vorliegenden Fall eine Laufbahn 147 bildet, wiederum zwei stationäre, also am Basisstandort 73 und an einem Nebenstandort 74 angeordnete Vermessungsvorrichtungen 70, 71 vorgesehen. Die Vermessungsvorrichtung 72 wird in diesem Fall nicht benötigt, da nur in der bestimmten Fläche 23 der Laufbahn 147 die Position des Objektes 2 bestimmt werden soll.

Die Ausbildung der Vermessungsvorrichtungen 70 und 71 entspricht denjenigen, wie sie in Fig. 4 gezeigt und beschrieben sind.

Die Positionsmeßeinrichtung 1 aus den Vermessungsvorrichtungen 70 und 71 dient dazu, beispielsweise zwischen den einzelnen Teilstrecken 148 bis 157 die Geschwindigkeit des Läufers, d.h. des Objektes 2, festzustellen.

Dazu ist ein Läufer 158 in verschiedenen Positionen 159 bis 170, beispielsweise während einer Fortbewegung von der Position 159 bis zur Position 170, gezeigt.

Um die Beschleunigung des Läufers 158 in den einzelnen Teilstrecken 148 bis 157 überwachen zu können, wird die Positionsmeßeinrichtung 1 außerhalb der Laufbahn 147 positioniert. Dazu wird mit den Sendern 78 und 82 der Vermessungsvorrichtungen 70, 71 eine Energiesendestrahlung 79 und 83 ausgesandt. Der Läufer 158 ist wiederum mit einem Positionsmelder 5 ausgestattet, die beim Eintreffen der Energiesendestrahlung 79 und 83 mit dem Identifikationscode 30 über den Empfänger 15 empfängt und den Identifikationscode 30 mit dem im Speicher 39 des Positionsmelders 5 vergleicht und über den Sender 16 die Energierücksendestrahlung 81 und 85 absendet.

Trifft nun die Energierücksendestrahlung 81 und 85 in der Vermessungsvorrichtung 70, 71 ein, wird sie wiederum der Steuereinrichtung 93 zugeführt. Die Steuereinrichtung 93 ist mit der Steuereinrichtung 93 in Fig. 4 vergleichbar, jedoch beinhaltet sie nicht mehr drei Sender 78, 82 und 86, sondern nur mehr die Sender 78 und 82. Die Berechnung der tatsächlichen Zeitdauer der Energierücksendestrahlungen 81 und 85 erfolgt wie in den zuvor beschriebenen Figuren.

Dabei entspricht die Zeit ta der Entfernung 172 und die Zeit tb der Entfernung 173.

Weiters ist in diesem Ausführungsbeispiel ersichtlich, daß die Feststellung der Position 159 bis 170 des Läufers 158 auch außerhalb der Laufbahn 147 festgestellt werden kann. Dies ist aus der Position 170 des Läufers 158 ersichtlich. Um diese Position 170 errechnen zu können, muß nun die Steuereinrichtung 93 die einzelnen Laufzeiten der Energierücksendestrahlungen 81 und 85 sowie die Distanz 76 zwischen den Vermessungsvorrichtungen 70 und 71 vergleichen. Durch diesen Vergleich kann nun die Steuereinrichtung 93 durch Feststellen des längsten Weges zwischen den Vermessungsvorrichtungen 70 und 71 ihre Bezugslinie als die Hypotenuse für das Dreieck zur Berechnung der Position 170 ermitteln.

Die Formeln für die Berechnung der Laufzeiten der Energierücksendestrahlungen 81 und 85 sowie die Zeit einer Entfernung 174 der die Distanz 76 zugeordnet ist, die das Kurzzeichen tc erhält, können die in Verbindung mit den zuvor beschriebenen Figuren angegebenen Formeln verwendet werden. Anhand der nachfolgenden Tabelle ist nun ein Berechnungsbeispiel für die Beschleunigung und die Geschwindigkeit des Läufers 158 dargestellt.

| Position | ta/ns | tb/ns | tc/ns |
|---|---|---|---|
| 159 | 51.666 | 337.330 | 333.333 |
| 160 | 56.033 | 315.933 | 333.333 |
| 161 | 71.200 | 289.000 | 333.333 |
| 162 | 95.233 | 258.533 | 333.333 |
| 163 | 123.633 | 226.966 | 333.333 |
| 164 | 153.933 | 195.300 | 333.333 |
| 165 | 185.033 | 164.033 | 333.333 |
| 166 | 216.266 | 133.733 | 333.333 |
| 167 | 248.100 | 104.366 | 333.333 |
| 168 | 279.800 | 77.933 | 333.333 |
| 169 | 311.633 | 57.833 | 333.333 |
| 170 | 342.900 | 51.000 | 333.333 |

Aus den gemessenen Laufzeiten ergeben sich folgende Strecken:

| Position | la/m | lb/m | lc/m |
|---|---|---|---|
| 159 | 15.499 | 101.190 | 100.000 |
| 160 | 16.809 | 94.779 | 100.000 |
| 161 | 21.360 | 86.700 | 100.000 |
| 162 | 28.569 | 77.559 | 100.000 |

(fortgesetzt)

| Position | la/m | lb/m | lc/m |
|---|---|---|---|
| 163 | 37.089 | 68.089 | 100.000 |
| 164 | 46.179 | 58.590 | 100.000 |
| 165 | 55.509 | 49.209 | 100.000 |
| 166 | 64.879 | 40.119 | 100.000 |
| 167 | 74.430 | 31.309 | 100.000 |
| 168 | 83.940 | 23.379 | 100.000 |
| 169 | 93.489 | 17.349 | 100.000 |
| 170 | 102.870 | 15.300 | 100.000 |

Daraus ergeben sich folgende Abstände relativ zur Vermessungsvorrichtung 70 und 71:

| Position | $lc_1$/m | h/m |
|---|---|---|
| 159 | 0.004 | 15.499 |
| 160 | 6.496 | 15.503 |
| 161 | 14.696 | 15.500 |
| 162 | 24.003 | 15.494 |
| 163 | 33.697 | 15.497 |
| 164 | 43.498 | 15.505 |
| 165 | 53.298 | 15.511 |
| 166 | 62.998 | 15.508 |
| 167 | 72.797 | 15.502 |
| 168 | 82.496 | 15.499 |
| 169 | 92.196 | 15.496 |
| 170 | 101.740 | 15.200 |

Mit diesen Werten ist nun die Position 159 bis 170 des bewegten Objektes 2 relativ zur Vermessungsvorrichtung 70 und der Vermessungsvorrichtung 71 festgelegt.

| Position | s/m |
|---|---|
| 159-160 | 6.492 |
| 160-161 | 8.200 |
| 161-162 | 9.306 |
| 162-163 | 9.693 |
| 163-164 | 9.801 |
| 164-165 | 9.799 |
| 165-166 | 9.700 |
| 166-167 | 9.798 |

(fortgesetzt)

| Position | s/m |
|---|---|
| 167-168 | 9.698 |
| 168-169 | 9.700 |
| 169-170 | 9.548 |
| 159-170 | 101.735 |

Die Objektgeschwindigkeit auf den Teilstrecken 148 bis 157 beträgt daher:

| Position | v/m/s | v/km/h |
|---|---|---|
| 159-160 | 6.492 | 23.371 |
| 160-161 | 8.200 | 29.520 |
| 161-162 | 9.306 | 33.501 |
| 162-163 | 9.693 | 34.894 |
| 163-164 | 9.801 | 35.283 |
| 164-165 | 9.799 | 35.276 |
| 165-166 | 9.700 | 34.920 |
| 166-167 | 9.798 | 35.272 |
| 167-168 | 9.698 | 34.912 |
| 168-169 | 9.700 | 34.920 |
| 169-170 | 9.548 | 34.372 |
| 159-170 | 9.248 | 33.292 |

Die Objektbeschleunigung auf den Teilstrecken beträgt somit

| Position | $v/m/s^2$ |
|---|---|
| 12-23 | 6.149 |
| 23-34 | 3.981 |
| 34-45 | 1.393 |
| 45-56 | 0.389 |
| 56-67 | -0.007 |
| 67-78 | -0.356 |
| 78-89 | 0.352 |
| 89-910 | -0.360 |
| 910-1011 | 0.008 |
| 1011-1112 | -0.548 |

In Fig. 7 ist eine weitere erfindungsgemäße Ausbildungsvariante der Positionsmeßeinrichtung 1 mit einer Vermessungsvorrichtung 175, z. B. zum Überwachen von Einsatzkräften bzw. Mitarbeitern an Einsatzorten oder in Firmen schematisch dargestellt. Die Vermessungsvorrichtung 175 beinhaltet wiederum einen Sender 176 und einen Empfänger 177. Der Sender 176 der Vermessungsvorrichtung 175 sendet eine Energiesendestrahlung 178, die als Richtstrahl 179 ausgebildet ist, aus. Die von zumindest einem Positionsmelder 5 abgesendete Energierücksendestrahlung 180 ist durch elektromagnetische Wellen gebildet, die sich kreisförmig von dem Positionsmelder 5 ausbreiten.

Der Positionsmelder 5 ist, wie bereits anhand der Übersichtsdarstellung in Fig. 1 gezeigt, ausgeführt.

Die Vermessungsvorrichtung 175 ist über Leitungen 181 bis 183 mit einer Steuereinrichtung 184 verbunden. Für die Aufnahme der Auswertung der Daten zumindest eines Positionsmelders 5 an einer Person 185 ist die Steuereinrichtung 184, die durch eine Rechnereinheit 186 gebildet sein kann, vorgesehen. In diesem Ausführungsbeispiel soll nun eine Ortung einer Person 185 dargestellt werden. Dabei sind an der Person 185 bzw. weiteren Personen 187, 188 Positionsmelder 5, 189, 190 angeordnet.

Die Rechnereinheit 186 wird über ein Eingabegerät 191 angesteuert. Dabei werden von der Rechnereinheit 186 die von dem Eingabegerät 191 eingegebenen Daten auf eine Ausgabegerät 192. das z.B. als Display-Anzeige oder Bildschirm ausgebildet sein kann, angezeigt. Zur Herstellung der Energiesendestrahlung 178, also dem Richtstrahl 179 und der Auswertung der Energierücksendestrahlung 180 ist die Rechnereinheit 186 mit einem Sendemodul 193, einem Empfangsmodul 194 und einem Winkelgeber 195 ausgestattet. Das Sendemodul 193, das Empfangsmodul 194 und ein Winkelgeber 195 sind über die Leitungen 181 bis 183 mit der Vermessungsvorrichtung 175 verbunden.

Weiters weist die Rechnereinheit 186 für das Sendemodul 193 eine Codiereinheit 29, wie in Fig. 1 dargestellt, auf, mit dem die Energiesendestrahlung 178 vor einem entsprechenden Identifikationscode 30 überlagert wird. Dieser Identifikationscode 30 ermöglich es, jeweils die einzelnen Positionsmelder 5, 189, 190 an der Person 185, 187, 188 anzusprechen, sodaß deren Bewegung von der in vollen Linien gezeichneten Stellung in die in strichlierten Linien gezeichnete Stellung überwacht werden kann.

Dabei ist in diesem Ausführungsbeispiel zu beachten, daß die Positionsmeßeinrichtung 1 aus einer Vermessungsvorrichtung 175 mit einem Sender 176 besteht, der einen Richtstrahl 179 über eine vorbestimmte Fläche 23 aussendet. Um die Position der Positionsmelder 5, 189, 190 zu erfassen, muß der Sender 176 der Vermessungsvorrichtung 175 nach Ablauf einer gewissen Zeitspanne 131, wie sie in Fig. 5 dargestellt ist, um einen bestimmten Winkel weiter gedreht werden. Dazu ist in der Steuereinrichtung 184 ein am Sender 176 befestigter Winkelgeber angeordnet. Dabei wird der Sender 176 der Vermessungsvorrichtung 175 um eine feststehende vertikale Schwenkachse 196 am Basisstandort 6 gedreht. Mit dem Winkelgeber 195 kann der Winkel der jeweiligen Drehstellung des Senders 176 festgestellt und der Rechnereinheit 186 übergeben werden.

Die Ausstrahlung der Energiesendestrahlung 178 erfolgt wie in den zuvor beschriebenen Figuren, wobei über die Codiereinheit 29 der Identifikationscode 30 und ein Startimpuls 56 bzw. Startsignal auf den Richtstrahl 179 moduliert ist. Dabei ist zu beachten, daß nach Ausstrahlung des Richtstrahls 179 eine gewisse Zeitspanne 131, die in der Rechnereinheit 186 voreinstellbar ist, abgewartet wird, bevor der Sender 176 um einen definierten Winkel weiter gedreht wird.

Trifft innerhalb der voreingestellten Zeitspanne 131 vom Positionsmelder 5, 189, 190 die Energierücksendestrahlung 180 am Empfangsmodul 194 ein, so wird der Zählerstand an eine Zählereinheit 197, die von einem Taktgenerator 198 angesteuert wird, abgefragt und der Rechnereinheit 186 zugeführt. Das Starten der Zählereinheit 197 erfolgt beim Aussenden des Startimpulses 56 bzw. Startsignals über den Richtstrahl 179.

Durch das Messen der Laufzeit vom Sender 176 bis zum jeweiligen Positionsmelder 5, 189, 190 und vom Positionsmelder 5, 189, 190 zurück zum Sender 176 kann die Entfernung 199 aus der Laufzeit der Energiesendestrahlung 178 und der Energierücksendestrahlung 180 wie in den zuvor beschriebenen Figuren errechnet werden. Aus der ermittelten Zeitdauer der Energiesende- und/oder Energierücksendestrahlung 178, 180 ist nunmehr die Distanz zwischen der Vermessungsvorrichtung 175 und dem jeweiligen Positionsmelder 5, 189, 190 bekannt. In Verbindung mit dem ebenfalls ermittelten Winkel 200 zwischen einer Bezugslinie 201 und der jeweiligen Position des Richtstrahls 179 während der Ermittlung der Zeitdauer bzw. Entfernung 199 kann nunmehr über trigonometrische Funktionen ähnlich der vorstehenden Beschreibung zu den anderen Ausführungsbeispielen wiederum die Position des Objektes 2 innerhalb der Fläche 23 ermittelt werden. Die Berechnung der einzelnen Entfernungen 199 zwischen der Vermessungsvorrichtung 175 und den einzelnen Personen 185, 187, 188 kann anhand eines entsprechenden Softwareprogrammes in der Rechnereinheit 186 erfolgen.

Mit einer derartigen Positionsmeßeinrichtung 1 ist es beispielsweise aber auch möglich, in der Rechnereinheit 186 die Positionen von entsprechend stationären Melde-und Empfangseinheiten, beispielsweise eines Telefons oder einer Gegensprechanlage abzuspeichern.

Wird nun eine Person 185, 187, 188 beispielsweise im Bereich eines Firmengeländes gesucht, so hat die jeweilige Dame beispielsweise in der Vermittlung lediglich die Codenummer für den jeweils angesprochenen Teilnehmer einzugeben. Über die Positionsmeßeinrichtung 1 kann darauf die Position der jeweiligen Person 185, 187, 188 sofort ermittelt und aus dieser Position festgestellt werden, wo sich die nächstgelegene Sende- und Empfangseinrichtung, beispielsweise die nächstgelegene Nebenstelle eines Telefons oder einer Gegensprechanlage, befindet und das Gespräch automatisch dort hingeleitet werden. Dadurch erübrigt sich die Mitnahme von teuren und aufwendigen Kommunikationseinrichtungen für die Mitarbeiter, und es kann damit eine größere Anzahl an Mitarbeitern überwacht werden. Die entsprechenden technischen Einrichtungen bzw. Schaltungen in Programmen zur Weiterleitung derartiger Anrufe und dergleichen sind aus der Fernmeldetechnik bekannt, und es können alle aus dem Stand der Technik bekannten Verfahren und Vorrichtungen hierfür eingesetzt werden, wenn die entsprechende Position über die erfindungsgemäße Positionsmeßeinrichtung 1 ermittelt ist.

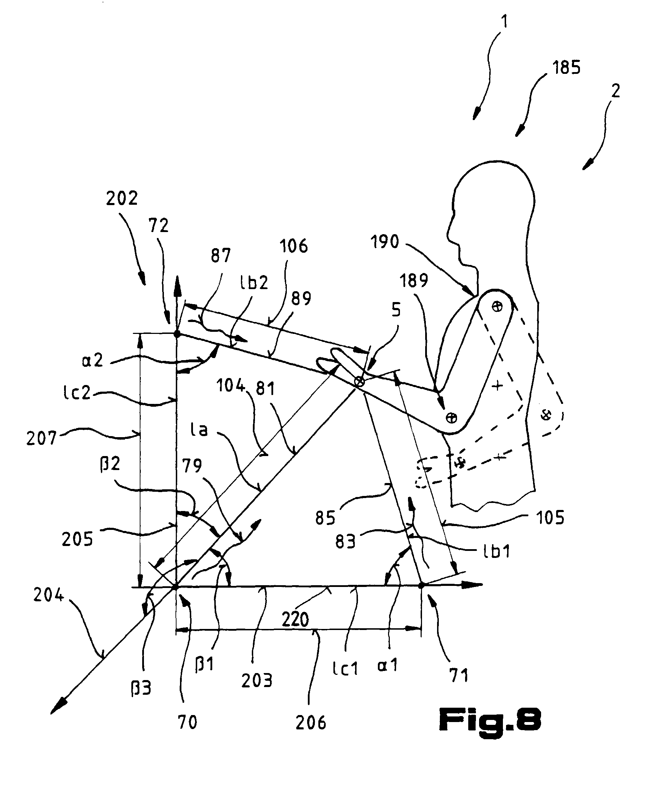

Anhand der Fig. 8 soll nun eine mögliche Ausführungsvariante der Positionsmeßeinrichtung 1 zur Ermittlung der Zeitdifferenz für ausgesendete bzw. empfangene Energiesendestrahlungen 79, 83 und 87 und Energierücksendestrahlungen 81, 85 und 89 zur Erfassung einer räumlichen Position des Objektes 2 in einer Fläche gezeigt und erläutert werden. Dabei ist in Fig. 8 ein karthesisches Koordinatensystem 202 mit einem bewegten Objekt 2, z. B. einer Person 185, gezeigt, die im Bereich mehrerer Gelenke Positionsmelder 5, 189, 190 angeordnet hat.

Die Ausbildung der Vermessungsvorrichtungen 70 bis 72 entspricht denjenigen, wie sie in Fig. 4 gezeigt sind, wobei die Darstellung der Steuereinrichtung 93 der besseren Übersichtlichkeit wegen in Fig. 8 nicht mehr dargestellt ist.

Die Positionsmeßeinrichtung 1 aus den Vermessungsvorrichtungen 70 bis 72 dient dazu, die Bewegungen der Person 185 räumlich darzustellen.

Die Vermessungsvorrichtungen 70, 71 und 72 sind zum Aufbau eines karthesischen Koordinatensystem 202 angeordnet. Das Koordinatensystem 202 wird durch die Achsen 203 bis 205 gebildet. Die Vermessungsvorrichtungen 70 bis 72 sind dabei in einer vorbestimmten Distanz. d.h. die Vermessungsvorrichtung 70 ist von der Vermessungsvorrichtung 71 in einer Entfernung 206 auf der Achse 203 und die Vermessungsvorrichtung 70 von der Vermessungsvorrichtung 72 in einer Entfernung 207 auf der Achse 204 angeordnet.

Die Entfernungen 104 bis 106 werden über das Zeitäquivalent der Laufzeit der Energiesendestrahlung 79, 83 und 87 und der Energierücksendestrahlung 81, 85 und 89 ermittelt, wobei die Ermittlung der Entfernungen 104 bis 106 in diesem Ausführungsbeispiel nicht mehr erläutert wird, da sie in den Fig. 3 und 4 bereits ausführlich erläutert wurde. Durch die Ermittlung der Entfernungen 104 bis 106 und 206, 207 durch die Rechnereinheit 95 ist nun eine Positionsberechnung für jeden der drei Positionsmelder 5, 189, 190 mit fünf gegebenen Abständen möglich, d.h. es kann ein dreidimensionales Bild erstellt werden.

Anhand eines Berechnungsbeispieles wird nun die Position des Objektes 2 berechnet. Dabei ergibt sich, daß die Entfernung $104 = la$, die Entfernung $105 = lb_1$, die Entfernung $106 = lb_2$, die Entfernung $206 = lc_1$ und die Entfernung $207 = lc_2$ entspricht.

Zur Vereinfachung der Berechnung wird der Ursprung des Koordinatensystems auf die Position der Vermessungsvorrichtung 70 gelegt. Die Achse 203 wird von der Vermessungsvorrichtung 70 durch die Vermessungsvorrichtung 71 gelegt und die Vermessungsvorrichtung 72 so angeordnet, daß die durch die Vermessungsvorrichtung 72 verlaufende Achse 205 einen rechten Winkel zu der Achse 203 einschließt. Die in der nachfolgenden Berechnung verwendeten Winkelbezeichnungen können aus der Zeichnung in Fig. 8 entnommen werden.

Aus dem Kosinussatz ergibt sich wie folgt:

$$\alpha 1 = \arccos\left(\frac{la^2 - lb_1^2 - lc_1^2}{-2 * lb_1 * lc_1}\right) \qquad \alpha 2 = \arccos\left(\frac{la^2 - lb_2^2 - lc_2^2}{-2 * lb_2 * lc_2}\right)$$

$$\beta 1 = \arccos\left(\frac{lb_1^2 - la^2 - lc_1^2}{-2 * la * lc_1}\right) \qquad \beta 2 = \arccos\left(\frac{lb_2^2 - la^2 - lc_2^2}{-2 * la * lc_2}\right)$$

Durch die aus der Vektorrechnung bekannte Beziehung

$$\cos^2\beta 1 + \cos^2\beta 2 + \cos^2\beta 3 = 1$$

erhält man:

$$\beta = \arccos\sqrt{(1 - \cos^2\beta 1 - \cos^2\beta 2)}$$

Da nun alle Winkel (β1,β2,β3) und der Abstand vom Nullpunkt, d.h. der Vermessungsvorrichtung 70 des Koordinatensystems (la) bekannt sind, kann man wie folgt den Vektor auf die Koordinatenachsen projizieren:

$x = la \cos β1$
$y = la \cos β3$
$z = la \cos β2$

Hat man nun die Koordinaten des Vektors errechnet, so läßt sich der Abstand zu einem anderem Vektor (z.B. vorherige Position) wie folgt berechnen.

$$s = \sqrt{(x_2 - x_1)^2 + (y_2 - y_1)^2 + (z_2 - z_1)^2}$$

Berechnung der momentanen Position des bewegten Objektes 2 aus der Laufzeit der Meßsignale zu drei Meßstationen der Vermessungsvorrichtungen 70 bis 72.

Bekannte Größen:

$ta$, $tb_1$, $tb_2$ ...... Laufzeit Strecke la, $lb_1$ und $lb_2$ (durch Laufzeitmessung ermittelte Abstände)
$tc_1$, $tc_2$ ........... Laufzeit Strecke $lc_1$ und $lc_2$ (durch Aufstellung der Vermessungsvorrichtungen 70 bis 72 bekannt)
c ..................... Lichtgeschwindigkeit c = 300 $10^6$ m/s

Strecken la, $lb_1$, $lb_2$, $lc_1$ und $lc_2$:

$$lx = tx * c$$

$ta = 50$ ns $\qquad la = 50 * 300\ 10^6 = 15.0$ m
$tb_1 = 56$ ns $\qquad lb_1 = 56 * 300\ 10^6 = 16.8$ m
$tb_2 = 60$ ns $\qquad lb_2 = 60 * 300\ 10^6 = 18$ m
$tc_1 = 70$ ns $\qquad lc_1 = 70 * 300\ 10^6\ ) = 21.0$ m
$tc_2 = 70$ ns $\qquad lc_2 = 70 * 300\ 10^6 = 21.0$ m

Winkel β1 und β2:

$$\beta = \arccos\left(\frac{lbx^2 - la^2 - lcx^2}{-2 * la * lcx}\right)$$

$$\beta 1 = \arccos\left(\frac{16{,}8^2 - 15^2 - 21^2}{-2 * 15 * 21}\right) = 52{,}47°$$

$$\beta 2 = \arccos\left(\frac{18^2 - 15^2 - 21^2}{-2 * 15 * 21}\right) = 57{,}12°$$

Winkel β3:

$$\beta 3 = \arccos \sqrt{(1 - \cos^2 \beta 1 - \cos^2 \beta 2)}$$

$$\beta 3 = \arccos \sqrt{(1 - \cos^2 52{,}47 - \cos^2 57{,}12)} = 54{,}68°$$

Koordinaten x, y und z:

x = la cos β1      x = 15 cos 52.47 = 9.13 m
y = la cos β3      y = 15 cos 54.68 = 8.67 m
z = la cos β2      z = 15 cos 57.12 = 8.14 m

Mit diesen Werten ist nun die Position des bewegten Objektes relativ zu den Vermessungsvorrichtungen 70 bis 72 bestimmt. Zwar würde ein Objekt 2, das an der Achse 203 gespiegelt wird, dieselben Meßergebnisse aufweisen, aber durch eine geeignete Aufstellung der Vermessungsvorrichtungen 70 bis 72, eine Bereichsvereinbarung (Spielfeldgrenze) festlegbar und unter Berücksichtigung der vorstehenden Ausführungen kann dieser Bereich ausgeschlossen werden.

Zur Berechnung des vom Objekt 2 zurückgelegten Weges wird nun folgendermaßen vorgegangen.

Bekannte Werte:

P1(x1,y1,z1), P2 (x2,y2,z2)

x1 = 9.13 m      x2 = 8.05 m
y1 = 8.67 m      y2 = 9.75 m
z1 = 8.14 m      z2 = 7.02 m

$$s = \sqrt{(x_2 - x_1)^2 + (y_2 - y_1)^2 + (z_2 - z_1)^2}$$

$$s = \sqrt{(8{,}05 - 9{,}13)^2 + (9{,}75 - 8{,}67)^2 + (7{,}02 - 8{,}14)^2} = 1{,}89\,m$$

Die Geschwindigkeits- und Beschleunigungsberechnungen erfolgen wie bei den zuvor beschriebenen zweidimensionalen Beispielen. Eine derartige Ausbildung der Positionsmeßeinrichtung 1 kann vor allem dazu verwendet werden, um beispielsweise die mechanischen Vorgänge, wie die Bewegung von Personen und/oder Tieren in der Verhaltensforschung bzw. für die Bewegungskoordination oder die Überwachung von Bewegungsabläufen bei Sportlern aufzuzeigen. So ist es möglich, die Positionsmelder 5, 189, 190 oder auch jede beliebige andere Anzahl solcher Positionsmelder an verschiedenen Körperteilen von Menschen oder Tieren anzuordnen, beispielsweise im Bereich der Gelenke oder im Bereich des Ober- und Unterschenkels, des Fußes und dergleichen.

Selbstverständlich können unter Verwendung einer derart ausgebildeten Positionsmeßeinrichtung bzw. entsprechend dem zuvor geschilderten Verfahrensverlauf auch Bewegungen von Maschinenteilen an unterschiedlichsten Maschinen, beispielsweise Baumaschinen, Kränen, Fertigungsautomaten und dergleichen überwacht werden.

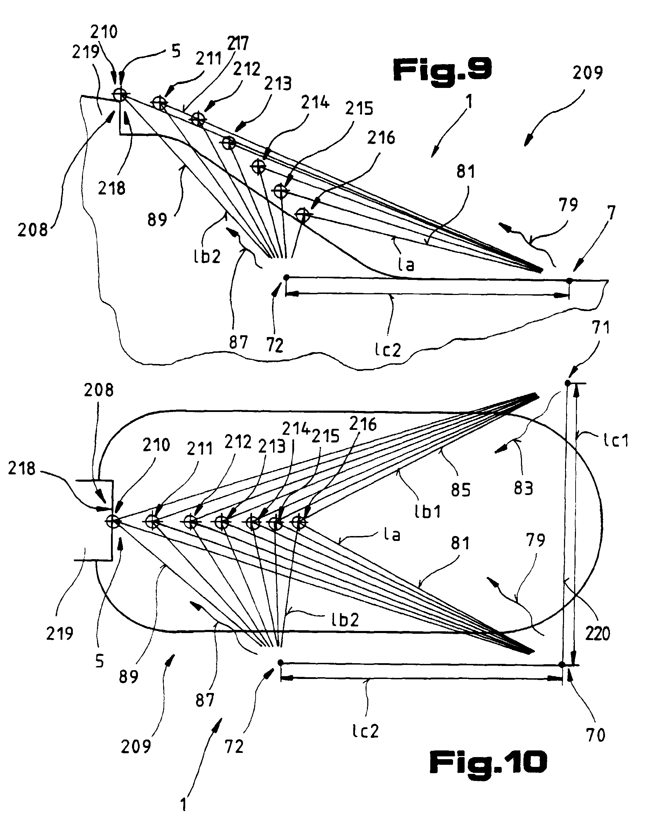

In den Fig. 9 und 10 ist eine mögliche Anwendungsvariante der Positionsmeßeinrichtung 1 nach Fig. 8 gezeigt.

Die Ausbildung der Vermessungsvorrichtungen 70 bis 72 entspricht den in Fig. 8 gezeigten.

Die Positionsmeßeinrichtung 1 aus den Vermessungsvorrichtungen 70 bis 72 dient dazu, beispielsweise die Sprungweite eines Schispringers 208, an dem ein Positionsmelder 5 befestigt ist, auf einer Schanzenanlage 209 zu bestimmen. Dazu entspricht die Schanzenanlage 209 der zu überwachenden Fläche 23.

Die Ermittlung der einzelnen Positionen 210 bis 216 erfolgt wieder über die Laufzeitermittlung der Energiesendestrahlungen 79, 83 und 87 und den Energierücksendestrahlungen 81, 85 und 89, wodurch wiederum die Entfernungen 104 bis 106 errechnet werden können.

Die Berechnung der Positionen 210 bis 216 kann nun anhand der Berechnungsweise insbesondere der Formeln, wie sie in Fig. 9 beschrieben sind, erfolgen.

Zur näheren Erläuterung des weiteren Berechnungsbeispieles anhand der in Fig. 9 und 10 gezeigten verschiedenen Positionen des Schispringers 208 wurden nunmehr folgende Werte für die Zeitdauer 144 => ta, die Zeitdauer 145 => $tb_1$, und die Zeitdauer 146 => $tb_2$ bemessen. Die Zeitdauer $tc_1$ und $tc_2$ errechnet sich aus der Differenz der Zeitmessungsergebnisse der Vermessungsvorrichtungen 70, 72 und den Vermessungsvorrichtungen 70, 71.

| Position | ta/ns | $tb_1$/ns | $tb_2$/ns | $tc_1$/ns | $tc_2$/ns |
|---|---|---|---|---|---|
| 210 | 1007.747 | 1007.747 | 583.095 | 533.333 | 533.333 |
| 211 | 946.279 | 946.279 | 539.114 | 533.333 | 533.333 |
| 212 | 880.183 | 880.383 | 492.383 | 533.333 | 533.333 |
| 213 | 810.408 | 810.408 | 442.558 | 533.333 | 533.333 |
| 214 | 740.353 | 740.353 | 390.000 | 533.333 | 533.333 |
| 215 | 672.252 | 672.525 | 337.154 | 533.333 | 533.333 |
| 216 | 614.336 | 614.336 | 289.811 | 533.333 | 533.333 |

Aus den gemessenen Laufzeiten errechnen sich folgende Strecken:

| Position | la/m | $lb_1$/m | $lb_2$/m | $lc_1$/m | $lc_1$/m |
|---|---|---|---|---|---|
| 210 | 302.324 | 302.324 | 174.928 | 160.000 | 160.000 |
| 211 | 283.883 | 283.883 | 161.734 | 160.000 | 160.000 |
| 212 | 264.054 | 264.054 | 147.715 | 160.000 | 160.000 |
| 213 | 243.122 | 243.122 | 132.767 | 160.000 | 160.000 |
| 214 | 222.105 | 222.105 | 117.000 | 160.000 | 160.000 |
| 215 | 210.675 | 201.675 | 101.146 | 160.000 | 160.000 |
| 216 | 184.301 | 184.301 | 86.943 | 160.000 | 160.000 |

Daraus können folgende Winkel berechnet werden:

| Position | β1/° | β2/° | β3/° |
|---|---|---|---|
| 210 | 74.655 | 26.736 | 68.663 |
| 211 | 73.631 | 28.237 | 67.663 |
| 212 | 72.364 | 29.552 | 67.095 |
| 213 | 70.788 | 30.431 | 67.352 |
| 214 | 68.888 | 30.495 | 69.054 |
| 215 | 66.629 | 29.728 | 72.688 |
| 216 | 64.273 | 28.134 | 79.384 |

Daraus können folgende Koordinatenwerte relativ zur Vermessungsvorrichtung 70 ermittelt werden:

| Position | x/m | y/m | z/m |
|----------|--------|---------|---------|
| 210 | 80.004 | 270.002 | 110.001 |
| 211 | 80.004 | 250.100 | 107.890 |
| 212 | 80.000 | 229.702 | 102.770 |
| 213 | 80.002 | 209.629 | 93.618 |
| 214 | 80.000 | 191.381 | 79.399 |
| 215 | 80.001 | 175.132 | 60.013 |
| 216 | 80.002 | 162.525 | 33.953 |

Mit diesen Werten ist nun die Position 210 bis 216 des bewegten Objektes 2 relativ zur Vermessungsvorrichtung 70 und der Vermessungsvorrichtung 71 und 72 bestimmt.

Aus diesen Werten ergibt sich die in den einzelnen Meßintervallen zurückgelegte Strecke:

| Position | s/m |
|----------|--------|
| 210-211 | 20.013 |
| 211-212 | 21.030 |
| 212-213 | 22.060 |
| 213-214 | 23.133 |
| 214-215 | 25.295 |
| 215-216 | 28.949 |

Die Objektgeschwindigkeit auf den einzelnen Teilstrecken beträgt:

| Position | v/m/s | v/km/h |
|----------|--------|--------|
| 210-211 | 2.013 | 2.046 |
| 11-212 | 21.030 | 75.708 |
| 212-213 | 22.060 | 79.416 |
| 213-214 | 23.133 | 83.278 |
| 214-215 | 25.295 | 91.062 |

Die Objektbeschleunigung auf den Teilstrecken beträgt somit:

| Position | v/m/s$^2$ | |
|----------|--------|---|
| 210-211 | 3.662 | |
| 211-212 | 3.708 | |
| 212-213 | 3.862 | |

**24**

(fortgesetzt)

| Position | v/m/s$^2$ | |
|----------|-----------|----------|
| 213-214 | 7.784 | |
| 214-215 | 13.154 | |
| 215-216 | 28.949 | 104.216 |

Da die Flugkurve eines Schispringers 208 eine räumliche Kurve darstellt, ist es in diesem Fall ebenso notwendig, wie in der Vermessungsvorrichtung 70 und 72 die weitere Vermessungsvorrichtung 71 anzuordnen, um feststellen zu können, ob sich die Positionen 210 bis 216 oberhalb oder unterhalb einer durch eine Bezugslinie 217 gebildeten direkten Verbindungslinie zwischen den Vermessungsvorrichtungen 70 bzw. 72 und einem Abspringpunkt 218 am Schanzentisch 219 der Schanzenanlage 209 befindet.

Auf die Anordnung einer vierten Vermessungsvorrichtung kann bei dieser Ausführungsform und Anordnungsvariante auf die erfindungsgemäße Positionsmeßeinrichtung 1 verzichtet werden, da sich der Meßbereich lediglich zwischen der Verbindungsgeraden der Vermessungsvorrichtungen 70 und 72 und dem Schanzentisch 219 erstreckt und der über die Verbindungslinie der Vermessungsvorrichtungen 70, 72 gespiegelte Bereich bzw. die gespiegelte Fläche zur Meßwertermittlung nicht herangezogen wird.

Sollten Sprungweiten erzielt werden, die über diese Verbindungslinie 220 zwischen den Vermessungsvorrichtungen 70 und 72 hinausgehen, so müßte noch eine vierte Vermessungsvorrichtung vorgesehen werden, um festzustellen, ob sich im Bezug auf den Schanzentisch 219 die Position des Schispringers zwischen der Verbindungslinie 220 und dem Schanzentisch 219 oder auf der vom Schanzentisch 219 abgewendeten Seite der Verbindungslinie 220 liegt.

Selbstverständlich ist es möglich, daß für die Positionsmeßeinrichtung 1 die beliebige Steuervorrichtung und Steuerverfahren zur Auswertung der Laufzeit der Entfernungen der Beschleunigungen und Geschwindigkeiten verwendet werden. Hierfür können sowohl analoge als auch digitale Einrichtungen, beispielsweise selbstprogrammierbare Steuerungen oder Industriepersonalcomputer oder speziell konfigurierte Rechnereinheiren oder in derartige Personalcomputer eingesetzte Rechnerkarren Verwendung finden.

Selbstverständlich können die Vermessungsvorrichtungen jeweils auch nur einen Sender oder einen Empfänger umfassen, sodaß beispielsweise an einem Basisstandort und einem oder mehreren Nebenstandorten nur mit Sendern und auch weitere Nebenstandorte nur mit Empfängern ausgestattete Vermessungsvorrichtungen angeordnet sind.

Des weiteren wurden der besseren Übersichtlichkeit wegen in den verschiedenen Ausführungsbeispielen bei der Erläuterung der Berechnung der unteschiedlichen Positionen, nur einzelne der Positionen mit den zugehörigen Meßcodes versehen. Für die dazwischen liegenden Positionen oder die verbleibenden Positionen gelten dann die entsprechend äquivalenten Maßangaben.

Selbstverständlich können auch einzelne Verfahrensschritte oder einzelne Ausbildungsdetails der Vorrichtung eigenständige, erfindungsgemäße Lösungen bilden bzw. auch Kombinationen aus solchen Einzelmerkmalen aus unterschiedlichen Ausführungsbeispielen eigenständige, erfinderische Kombinationen ergeben.

Zur Darstellung der Funktion und des Verfahrensablaufes der erfindungsgemäßen Positionsmeßeinrichtung wurden die Größenordnungen teilweise stark verzerrt bzw. unmaßstäblich, sowie schematisch dargestellt, um das Verständnis für die erfindungsgemäßen Lösungen zu erleichtern.

Vor allem können die einzelnen, in den Fig. 1; 2,3; 4,5; 6; 7; 8; 9,10 gezeigten Ausführungen den Gegenstand von eigenständigen, erfindungsgemäßen Lösungen bilden. Die diesbezüglichen erfindungsgemäßen Aufgaben und Lösungen sind den Detailbeschreibungen dieser Figuren zu entnehmen.

**Bezugszeichenaufstellung**

| | |
|---|---|
| 1 | Positionsmeßeinrichtung |
| 2 | Objekt |
| 3 | Vermessungsvorrichtung |
| 4 | Vermessungsvorrichtung |
| 5 | Positionsmelder |
| 6 | |
| 7 | |
| 8 | Basisstandort |

| 9 | Nebenstandort |
|---|---|
| 10 | Distanz |
| | Sender |
| 11 | Energiesendestrahlung |
| 12 | |
| 13 | Empfänger |
| 14 | Energierücksendestrahlung |
| 15 | Empfänger |
| | Energierücksendestrahlung |
| 16 | Empfänger |
| 17 | |
| 18 | Sender |
| 19 | Decodiereinheit |
| 20 | Stromquelle |
| | Leitung |
| 21 | Leitung |
| 22 | |
| 23 | Leitung |
| 24 | Steuereinrichtung |
| 25 | Fläche |
| | Rechnereinheit |
| 26 | Eingabegerät |
| 27 | |
| 28 | Sendereinheit |
| 29 | Empfängereinheit |
| 30 | Empfängereinheit |
| | Codiereinheit |
| 31 | Identifikationscode |
| 32 | |
| 33 | Entfernung |
| 34 | Entfernung |
| 35 | Taktgenerator |
| | Zählereinheit |
| 36 | Befehlseingabegerät |
| 37 | |
| 38 | Speicher |
| 39 | Ausstrahlungswinkel |
| 40 | Steuerleitung |
| | Speicher |
| | Lagerplatz |
| 41 | Abstellplatz |
| 42 | Abstellplatz |
| 43 | Ware |
| 44 | Ware |
| 45 | Hubstapler |
| 46 | Position |
| 47 | Position |
| 48 | Position |
| 49 | Position |
| 50 | |
| 51 | Frequenz |
| 52 | Zeitpunkt |
| 53 | Startimpuls |
| 54 | Amplitude |
| 55 | Amplitude |
| 56 | Startimpuls |
| 57 | Zeitspanne |
| 58 | Zeitpunkt |

| | |
|---|---|
| 59 | Grundlinie |
| 60 | Grundlinie |
| 61 | Grundlinie |
| 62 | Amplitude |
| 63 | Frequenz |
| 64 | Zeitpunkt |
| 65 | Zeitpunkt |
| 66 | Zeitdauer |
| 67 | Zeitdauer |
| 68 | Zeitdauer |
| 69 | Zeitdauer |
| 70 | Vermessungsvorrichtung |
| 71 | Vermessungsvorrichtung |
| 72 | Vermessungsvorrichtung |
| 73 | Basisstandort |
| 74 | Nebenstandort |
| 75 | Nebenstandort |
| 76 | Distanz |
| 77 | Abstand |
| 78 | Sender |
| 79 | Energiesendestrahlung |
| 80 | Empfänger |
| 81 | Energierücksendestrahlung |
| 82 | Sender |
| 83 | Energiesendestrahlung |
| 84 | Empfänger |
| 85 | Energierücksendestrahlung |
| 86 | Sender |
| 87 | Energiesendestrahlung |
| 88 | Empfänger |
| 89 | Energierücksendestrahlung |
| 90 | Leitung |
| 91 | Leitung |
| 92 | Leitung |
| 93 | Steuereinrichtung |
| 94 | Fläche |
| 95 | Rechnereinheit |
| 96 | Eingabegerät |
| 97 | Sendemodul |
| 98 | Sendemodul |
| 99 | Sendemodul |
| 100 | Empfängermodul |
| 101 | Codiermodul |
| 102 | Codiermodul |
| 103 | Codiermodul |
| 104 | Entfernung |
| 105 | Entfernung |
| 106 | Entfernung |
| 107 | Taktgenerator |
| 108 | Zählereinheit |
| 109 | Position |
| 110 | Position |
| 111 | Position |
| 112 | Position |
| 113 | Position |
| 114 | Position |
| 115 | Position |
| 116 | Position |

117 Position

118 Speicher

119 Decodiermodul

120 Grundlinie

121 Grundlinie

122 Grundlinie

123 Grundlinie

124 Grundlinie

125 Grundlinie

126 Zeitpunkt

127 Startimpuls

128 Amplitude

129 Amplitude

130 Startimpuls

131 Zeitspanne

132 Zeitpunkt

133 Zeitpunkt

134 Zeitpunkt

135 Amplitude

136 Frequenz

137 Frequenz

138 Zeitpunkt

139 Zeitpunkt

140 Zeitpunkt

141 Zeitdauer

142 Zeitdauer

143 Zeitdauer

144 Zeitdauer

145 Zeitdauer

146 Zeitdauer

147 Laufbahn

148 Teilstrecke

149 Teilstrecke

150 Teilstrecke

151 Teilstrecke

152 Teilstrecke

153 Teilstrecke

154 Teilstrecke

155 Teilstrecke

156 Teilstrecke

157 Teilstrecke

158 Läufer

158 Position

160 Position

161 Position

162 Position

163 Position

164 Position

165 Position

166 Position

167 Position

168 Position

169 Position

170 Position

171

172 Entfernung

173 Entfernung

174 Entfernung

175 Vermessungsvorrichtung
176 Sender
177 Empfänger
178 Energiesendestrahlung
179 Richtstrahl
180 Energierücksendestrahlung
181 Leitung
182 Leitung
183 Leitung
184 Steuereinrichtung
185 Person
186 Rechnereinheit
187 Person
188 Person
189 Positionsmelder
190 Positionsmelder
191 Eingabegerät
192 Ausgabegerät
193 Sendemodul
194 Empfangsmodul
195 Winkelgeber
196 Schwenkachse
197 Zählereinheit
198 Taktgenerator
199 Entfernung
200 Winkel
201 Bezugslinie
202 Koordinatensystem
203 Achse
204 Achse
205 Achse
206 Entfernung
207 Entfernung
208 Schispringer
209 Schanzenanlage
210 Position
211 Position
212 Position
213 Position
214 Position
215 Position
216 Position
217 Bezugslinie
218 Abspringpunkt
219 Schanzentisch
220 Verbindungslinie

**Patentansprüche**

1. Verfahren zum Feststellen der Laufzeit einer abgesandten Energiesendestrahlung (10, 79, 83, 87, 178) zwischen einem Basisstandort (6, 73) und zumindest einem Positionsmelder (5, 189, 190) auf einem davon distanzierten beweglichen Objekt (2), insbesondere für eine Positionsbestimmung des Objektes (2), bei dem die Energiesendestrahlung (10, 79, 83, 87, 178) zumindest über eine dem Basisstandort (6, 73) benachbarte oder diesen umschließenden Fläche mit einer vorgegebenen Frequenz der Energiesendestrahlung (10, 79, 83, 87, 178) ausgesendet wird, worauf nach Eintreffen der Energiesendestrahlung (10, 79, 83, 87, 178) am Positionsmelder (5, 189, 190) von diesem eine Energierücksendestrahlung (12, 14, 81, 85, 89, 180) mit vorgegebener Frequenz der Energierücksendestrahlung (12, 14, 81, 85, 89, 180) ausgesandt wird, worauf am Basisstandort (6, 73) der Eingang dieser Energierücksendestrahlung (12, 14, 81, 85, 89, 180) festgestellt wird und die Laufzeit der Energiesendestrahlung (10, 79, 83, 87, 178) und der Energierücksendestrahlung (12, 14, 81, 85, 89, 180) zwischen dem Basisstandort (6, 73) und zumindest dem beweglichen Objekt (2) ermittelt wird, dadurch gekennzeichnet, daß auf die Energiesendestrahlung (10, 79, 83, 87, 178) ein Startimpuls (53, 56, 127, 130) bzw. ein Startsignal aufmoduliert wird, worauf nach Einlangen des Startimpulses (53, 56, 127, 130) bzw. des Startsignals am Positionsmelder (5, 189, 190) dieser aktiviert wird und von diesem die Energierücksendestrahlung (12, 14, 81, 85, 89, 180) ausgesandt wird, wobei auf die Energierücksendestrahlung (12, 14, 81, 85, 89, 180) ein Startimpuls bzw. ein Stoppimpuls aufmoduliert wird.

2. Verfahren nach Anspruch 1, dadurch gekennzeichnet, daß vom Basisstandort (6) ein die Energiesendestrahlung (178) bildender Richtstrahl (179) ausgesandt wird, der mit einem dem Schwenkwinkel gegenüber einer Bezugslinie (201) entsprechenden Identifikationscode (30) versehen ist und daß nach Ablauf einer voreinstellbaren maximalen Zeitspanne (131) eines Meßzyklus der Winkel (200) des Richtstrahls (179) gegenüber der Bezugslinie (201) verändert wird und daß beim Auftreffen des Richtstrahls (179) auf einen Positionsmelder (5, 189, 190) eine Energierücksendestrahlung (180) ausgelöst wird und die Zeitdauer der Übermittlung vom Basisstandort (6, 73) zum Objekt (2) und zurück zum Basisstandort (6, 73) ermittelt wird.

3. Verfahren nach Anspruch 2, dadurch gekennzeichnet, daß der Winkel (200) des Richtstrahls (179) gegenüber einer Bezugslinie (201) bei Absendung eines Identifikationscodes (30) ermittelt und falls eine Zeitdauer in diesem Meßzyklus ermittelt wird, dieser Zeitdauer zugeordnet wird.

4. Verfahren nach Anspruch 2 oder 3, dadurch gekennzeichnet, daß ein Sender (176) einer Vermessungsvorrichtung (175) am Basisstandort (6, 73) um eine winkelig zur überwachenden Fläche (23) verlaufende Schwenkachse (196) zwischen die äußersten Endpunkte der Fläche (23) bestimmenden Winkelbegrenzenden taktweise um einen voreinstellbaren Bruchteil des Gesamtwinkels verschwenkbar ist und dieTaktzeit durch die maximale Zeitspanne (131) des Meßzyklus beschränkt ist.

5. Verfahren nach einem oder mehreren der Ansprüche 1 bis 4, dadurch gekennzeichnet, daß die Energierücksendestrahlung (12, 14) am Basisstandort (6) und einem weiteren von diesem in einer voreinstellbaren oder überwachbaren Distanz (8) angeordneten Nebenstandort (7) festgestellt wird.

6. Verfahren nach einem oder mehreren der Ansprüche 1 bis 5, dadurch gekennzeichnet, daß die Sende- und/oder Abtastvorgänge am Basisstandort (6) und am Nebenstandort (7) sowie gegebenenfalls die Messung der Distanz (8) zwischen diesen beiden zeitsynchronisiert sind.

7. Verfahren nach einem oder mehreren der Ansprüche 1 bis 6, dadurch gekennzeichnet, daß die Energiesendestrahlung (10) mit einem Identifikationscode (30) versehen ist und nach dem Einlangen der Energiesendestrahlung (10) am Objekt (2) der Identifikationscode (30) der Energiesendestrahlung (10) und des Objektes (2) miteinander verglichen werden und die Energierücksendestrahlung (12, 14) nur bei Zusammengehörigkeit insbesondere Übereinstimmung der Identifikationscodes (30) abgesendet wird.

8. Verfahren nach einem oder mehreren der Ansprüche 1 bis 7, dadurch gekennzeichnet, daß nach dem Einlangen der Energiesendestrahlung (10) am beweglichen Objekt (2) mit der Energierücksendestrahlung (12, 14) ein Identifikationscode (30) des Objektes (2) mitübermittelt wird.

9. Verfahren nach einem oder mehreren der Ansprüche 1 bis 8, dadurch gekennzeichnet, daß von dem Basisstandort (6, 73) und zumindest einem Nebenstandort (7, 74, 75) jeweils eine Energiesendestrahlung (10, 79, 83, 87), gegebenenfalls mit einem Identifikationscode (30) ausgesendet wird und nur bei Zusammengehörigkeit der Identifikationscodes (30) der Energiesendestrahlung (10, 79, 83, 87) und des Objektes (2) jeweils nach dem Einlangen jeder Energiesendestrahlung (10, 79, 83, 87) eine gegebenenfalls mit einem Identifikationscode (30) versehene Energierücksendestrahlung (12, 14, 81, 85, 89) ausgesendet wird und im Basis- und Nebenstandort (73, 74, 75) nur die diesem zugeordnete, von dieser ausgesandten Energiesendestrahlung (10, 79, 83, 87) ausgelöste Energierücksendestrahlung (12, 14, 81, 85, 89) verarbeitet wird.

10. Verfahren nach einem oder mehreren der Ansprüche 1 bis 9, dadurch gekennzeichnet, daß die Frequenz (51, 136) der Energiesendestrahlung (10, 79, 83, 87) zu der Frequenz (63, 137) der Energierücksendestrahlung (12, 14, 81, 85, 89) unterschiedlich, insbesondere höher ist.

11. Verfahren nach einem oder mehreren der Ansprüche 5 bis 10, dadurch gekennzeichnet, daß der Basis- und der Nebenstandort (6, 73; 7, 74) in einer vorbestimmbaren Distanz (8, 76) angeordnet sind und die bevorzugt veränderbare Distanz (8, 76) zwischen dem Basis- und Nebenstandort (6, 73; 7, 74) bevorzugt über die Energiesendestrahlung (10, 79, 83, 87) ermittelbar und/oder überwachbar ist.

12. Verfahren nach einem oder mehreren der Ansprüche 5 bis 11, dadurch gekennzeichnet, daß aus der Distanz (8, 76) des Basis- und Nebenstandortes (6, 73, 7, 74) und der Laufdauer der Energiesende- (10, 79, 83, 87) und/oder Energierücksendestrahlung (12, 14, 81, 85, 89) in Abhängigkeit von der Ausbreitungsgeschwindigkeit der Strahlung eine Distanz (8, 76) zwischen dem Basis- und/oder Nebenstandort (6, 73, 7, 74) und dem beweglichen Objekt (2) erfaßt wird.

13. Verfahren nach einem oder mehreren der Ansprüche 2 bis 12, dadurch gekennzeichnet, daß der Identifikationscode (30) auf die Energiesendestrahlung (10, 79, 83, 87), z.B. durch Frequenzmodulation aufmoduliert ist.

14. Verfahren nach einem oder mehreren der Ansprüche 5 bis 13, dadurch gekennzeichnet, daß der auf die Energiesendestrahlung (10, 79, 83, 87) aufmodulierte Identifikationscode (30) einen Startimpuls (53, 56, 127, 130) bzw. Startsignal zum Beginn der Zeitfeststellung am Basis- (6, 73) und Nebenstandort (7, 74, 75) und/oder Bezugspunkt aufweist und die Energierücksendestrahlung (12, 14, 81, 85, 89) durch diesen Startimpuls (53, 56, 127, 130) ausgelöst wird.

15. Verfahren nach einem oder mehreren der Ansprüche 1 bis 14, dadurch gekennzeichnet, daß über die Energiesendestrahlung (10, 79, 83, 87) innerhalb einer Zeitspanne (57, 131) des maximalen Meßzyklus mehrere, zumindest zwei Identifikationscodes (30) und gegebenenfalls Startimpulse (53, 56, 127, 130) bzw. Startsignale und/oder Stoppcodes ausgesendet werden.

16. Verfahren nach einem oder mehreren der Ansprüche 1 bis 15, dadurch gekennzeichnet, daß mit einem Identifikationscode (30) mehrere Objekte (2) bzw. die auf den Objekten (2) angeordneten Positionsmelder (5, 189, 190) angesprochen werden, und die Energierücksendestrahlungen (12, 14, 81, 85, 89, 180) eine bevorzugt unterschiedliche Frequenz (63,137) bzw. unterschiedliche Identifikationscodes (30) und/oder Stopp- und/oder Startbefehle umfassen.

17. Verfahren nach einem oder mehreren der Ansprüche 1 bis 16, dadurch gekennzeichnet, daß die Ermittlung der Positionen der verschiedenen Objekte (2) in Echtzeit erfolgt.

18. Verfahren nach einem oder mehreren der Ansprüche 1 bis 17, dadurch gekennzeichnet, daß die Postionsmelder (5, 189, 190) auf verschiedenen Körperteilen einer Person (185) und/oder Tieren, insbesondere im Bereich von Gelenken derselben angeordnet sind.

**Claims**

1. Process for finding the time of flight of radiated energy (10, 79, 83, 87, 178) between a base (6, 73) and at least one position detector (5, 189, 190) on a moving object (2) at a distance therefrom, in particular for determining the position of the object (2), in which the radiated energy (10, 79, 83, 87, 178) is radiated at least over a surface adjacent to the base (6, 73) or enclosing the latter with a predetermined frequency for the radiated energy (10, 79, 83, 87, 178), after which after the impingement of the radiated energy (10, 79, 83, 87, 178) on the position detector (5, 189, 190) from the latter a returned energy (12, 14, 81, 85, 89, 180) with a predetermined frequency for the returned energy (12, 14, 81, 85 89, 180) is emitted, after which the arrival of said returned energy (12, 14, 81, 85, 89, 180) is established on the base (6, 73) and the time of flight of the radiated energy (10, 79, 83, 87, 178) and the returned energy (12, 14, 81, 85, 89, 180) between the base (6, 73) and at least the moving object (2) is determined, characterised in that a start pulse (53, 56, 127, 130) or a start signal is modulated onto the radiated energy (10, 79, 83, 87, 178), whereupon after the arrival of the start pulse (53, 56, 127, 130) or start signal at the position detector (5, 189, 190) the latter is activated and by the latter the returned energy (12, 14, 81, 85, 89, 180) is radiated, whereby a start pulse or a stop pulse is modulated onto the returned energy (12, 14, 81, 85, 89, 180).

2. Process according to claim 1, characterised in that from the base (6) a directional beam (179) forming the radiated energy (178) is emitted, which is provided with an identification code (30) corresponding to the angle relative to a reference line (201), and in that after the expiry of a prespecifiable maximum time span (131) of a measurement cycle the angle (200) of the directional beam (179) relative to the reference line (201) is changed, and in that on the impingement of the directional beam (179) on a position detector (5, 189, 190) a returned energy (180) is triggered, and the period of transmission from the base (6, 73) to the object (2) and back to the base (6, 73) is detected.

3. Process according to claim 2, characterised in that the angle (200) of the directional beam (179) relative to a reference line (201) on transmitting an identification code (30) is detected and if a period is detected in this measurement cycle is assigned to this period.

4. Process according to claim 2 or 3, characterised in that a transmitter (176) of a measuring device (175) on the base (6, 73) is pivotable about a pivot axis (196) running at an angle to the surface (23) to be monitored between the outermost end points of the angle limits determining the surface (23) in cycle by a prespecifiable fraction of the total angle and the cycle time is limited by the maximum time span (131) of the measurement cycle.

5. Process according to one or more of claims 1 to 4, characterised in that the returned energy (12, 14) is established on the base (6) and an additional secondary base (7) arranged at a prespecificable or monitorable distance (8) from the latter.

6. Process according to one or more of claims 1 to 5, characterised in that the transmission and/or scanning processes on the base (6) and on the secondary base (7) and if necessary the measurement of the distance (8) between the latter is time synchronised.

7. Process according to one or more of claims 1 to 6, characterised in that the radiated energy (10) is provided with an identification code (30) and after the arrival of the radiated energy (10) on the object (2) the identification code (30) of the radiated energy (10) and of the object (2) are compared and the returned energy (12, 14) is only transmitted if there is a correspondence in particular coincidence of the identification codes (30).

8. Process according to one or more of claims 1 to 7, characterised in that after the arrival of the radiated energy (10) on the moving object (2) with the returned energy (12, 14) an identification code (30) of the object (2) is also transmitted.

9. Process according to one or more of claims 1 to 8, characterised in that from the base (6, 73) and at least one secondary base (7, 74, 75) radiated energy (10, 79, 83, 87) is transmitted if necessary with an identification code (30), and only if there is a correspondence between the identification codes (30) of the radiated energy (10, 79, 83, 87) and the object (2) respectively after the arrival of each radiated energy (10, 79, 83, 87) is returned energy (12, 14, 81, 85, 89) provided if necessary with an identification code (30) transmitted, and in the base and secondary base (73, 74, 75) only the returned energy (12, 14, 81, 85, 89) assigned thereto and triggered by the emitted radiated energy (10, 79, 83, 87) is processed.

10. Process according to one or more of claims 1 to 9, characterised in that the frequency (51, 136) of the radiated energy (10, 79, 83, 87) is different from the frequency (63, 137) of the returned energy (12, 14, 81, 85, 89), in particular is higher.

11. Process according to one or more of claims 5 to 10, characterised in that the base and the secondary base (6, 73; 7, 74) are arranged at a predeterminable distance (8, 76), and the preferably variable distance (8, 76) between the base and secondary base (6, 73; 7, 74) is preferably detectable and/or monitorable by the radiated energy (10, 79, 83, 87).

12. Process according to one or more of claims 5 to 11, characterised in that from the distance (8, 76) between the base and secondary base (6, 73, 7, 74) and the time of flight of radiated energy (10, 79, 83, 87) and/or returned energy (12, 14, 81, 85, 89) depending on the propagation speed of the radiation a distance (8, 76) is detected between the base and/or secondary base (6, 73, 7, 74) and the moving object (2).

13. Process according to one or more of claims 2 to 12, characterised in that the identification code (30) is modulated onto the radiated energy (10, 79, 83, 87), e.g. by frequency modulation.

14. Process according to one or more of claims 5 to 13, characterised in that identification code (30) modulated onto the radiated energy (10, 79, 83, 87), has a start pulse (53, 56, 127, 130) or start signal for beginning the time detection on the base (6, 73) and secondary base (7, 74, 75) and/or reference point and the returned energy (12, 14, 81, 85, 89) is triggered by this start pulse (53, 56, 127, 130).

**15.** Process according to one or more of claims 1 to 14, characterised in that via the radiated energy (10, 79, 83, 87) within a time span (57, 131) of the maximum measurement cycle several, at least two identification codes (30) and if necessary start pulses (53, 56, 127, 130) or start signals and/or stop codes are emitted.

**16.** Process according to one or more of claims 1 to 15, characterised in that with an identification code (30) several objects (2) or the position detectors (5, 189, 190) arranged on the objects (2) are actuated and the returned energy (12, 14, 81, 85, 89, 180) has a preferably variable frequency (63, 137) or different identification codes (30) and/or stop and/or start commands.

**17.** Process according to one or more of claims 1 to 16, characterised in that the detection of the positions of the various objects (2) occurs in real time.

**18.** Process according to one or more of claims 1 to 17, characterised in that the position detectors (5, 189, 190) are arranged on different parts of the body of a person (185) and/or animals, particularly in the region of the joints thereof

## Revendications

**1.** Procédé pour déterminer le temps de propagation d'un rayonnement énergétique émis (10,79,83,87,178) entre un poste de base (6,73) et au moins un indicateur de position (5,189,190) sur un objet mobile (2) espacé de celui-ci, notamment pour une détermination de la position de l'objet (2), où le rayonnement énergétique (10,79,83,87,178) est émis au moins sur une surface avoisinant le poste de base (6,73) ou entourant celui-ci, avec une fréquence prédéterminée du rayonnement énergétique (10,79,83,87,178), où après l'arrivée du rayonnement énergétique (10,79,83,87,178) à l'indicateur de position (5,189,190) est émis par celui-ci un rayonnement énergétique de retour (12,14,81,85,89,180) d'une fréquence prédéterminée du rayonnement énergétique de retour (12,14,81,85,89,180), à la suite de quoi est constaté au poste de base (6,73) l'arrivée de ce rayonnement énergétique de retour (12,14,81,85,89,180) et le temps de propagation du rayonnement énergétique (10,79,83,87,178) et du rayonnement énergétique de retour (12,14,81,85,89,180) est déterminé entre le poste de base (6,73) et au moins l'objet mobile (2), caractérisé en ce qu'il est modulé sur le rayonnement énergétique (10,79,83,87,178) une impulsion de départ (53,56,127,130) respectivement un signal de départ, où, après l'arrivée de l'impulsion de départ (53,56,127,130) respectivement du signal de départ à l'indicateur de position (5,189,190) celui-ci est activé et le rayonnement énergétique de retour (12,14,81,85,89,180) est émis par celui-ci, où est modulé sur le rayonnement énergétique de retour (12,14,81,85,89,180) une impulsion de départ respectivement une impulsion d'arrêt.

**2.** Procédé selon la revendication 1, caractérisé en ce qu'il est émis à partir du poste de base (6) un faisceau dirigé (179) formant le rayonnement énergétique (178) qui est pourvu d'un code d'identification (30) correspondant à l'angle de pivotement par rapport à une ligne de référence (201) et qu'après l'écoulement d'un laps de temps maximal préréglable (131) d'un cycle de mesure, l'angle (200) du faisceau dirigé (179) est modifié par rapport à la ligne de référence (201), et que lors de l'incidence du faisceau dirigé (179) sur un indicateur de position (5,189,190), un rayonnement énergétique de retour (180) est déclenché et la durée de la transmission du poste de base (6,73) à l'objet (2) et de nouveau au poste de base (6,73) est déterminé.

**3.** Procédé selon la revendication 2, caractérisé en ce que l'angle (200) du faisceau dirigé (179) est déterminé par rapport à une ligne de référence (201) lors de l'émission d'un code d'identification (30) et, dans le cas où une durée est déterminée dans ce cycle de mesure, est associé à cette durée.

**4.** Procédé selon la revendication 2 ou 3, caractérisé en ce qu'un émetteur (176) d'un dispositif de mesurage (175) au poste de base (6,73) peut être amené à pivoter autour d'un axe de pivotement (196) s'étendant selon un angle à la surface à surveiller (23), entre les limiteurs angulaires définissant les points d'extrémité les plus extérieurs de la surface (23), d'une manière cadencée selon une fraction préréglable de l'angle total, et que la cadence est limitée par le laps de temps maximal (131) du cycle de mesure.

**5.** Procédé selon l'une ou plusieurs des revendications 1 à 4, caractérisé en ce que le rayonnement énergétique de retour (12,14) est constaté au poste de base (6) et à un poste secondaire supplémentaire (7) disposé par rapport à celui-ci à une distance (8) préréglable ou pouvant être surveillée.

**6.** Procédé selon l'une ou plusieurs des revendications 1 à 5, caractérisé en ce que les opérations d'émission et/ou de balayage sont synchronisées dans le temps au poste de base (6) et au poste secondaire (7) ainsi que le cas échéant la mesure de la distance (8) entre les deux postes précités.

7. Procédé selon l'une ou plusieurs des revendications 1 à 6, caractérisé en ce que le rayonnement énergétique (10) est pourvu d'un code d'identification (30) et qu'après l'arrivée du rayonnement énergétique (10) à l'objet (2), le code d'identification (30) du rayonnement énergétique (10) et de l'objet (2) sont comparés l'un avec l'autre et que le rayonnement énergétique de retour (12,14) est émis seulement lors d'une parentée notamment coïncidence des codes d'identification (30).

8. Procédé selon l'une ou plusieurs des revendications 1 à 7, caractérisé en ce qu'après l'arrivée du rayonnement énergétique (10) à l'objet mobile (2), il est transmis avec le rayonnement énergétique de retour (12,14) un code d'identification (30) de l'objet (2).

9. Procédé selon l'une ou plusieurs des revendications 1 à 8, caractérisé en ce qu'il est émis par le poste de base (6,73) et au moins un poste secondaire (7,74,75) respectivement un rayonnement énergétique (10,79,83,87), le cas échéant avec un code d'identification (30) et que seulement lors d'une parentée du code d'identification (30) du rayonnement énergétique (10,79,83,87) et de l'objet (2), respectivement après l'arrivée de chaque rayonnement énergétique (10,79,83,87), il est émis un rayonnement énergétique de retour (12,14,81,85,89) pourvu le cas échéant d'un code d'identification (30) et qu'il est traité dans les postes de base et secondaires (73,74,75) seulement le rayonnement énergétique de retour (12,14,81,85,89) associé à celui-ci, déclenché par ce rayonnement énergétique émis (10,79,83,87).

10. Procédé selon l'une ou plusieurs des revendications 1 à 9, caractérisé en ce que la fréquence (51,136) du rayonnement énergétique (10,79,83,87) est différente de la fréquence (63,137) du rayonnement énergétique de retour (12,14,81,85,89), notamment plus élevée que celle-ci.

11. Procédé selon l'une ou plusieurs des revendications 5 à 10, caractérisé en ce que les postes de base et secondaires (6,73;7,74) sont disposés à une distance apte à être prédéterminée (8,76), et que la distance (8,76) de préférence modifiable peut être déterminée et/ou surveillée entre les postes de base et secondaires (6,73;7,74) de préférence par le rayonnement énergétique (10,79,83,87).

12. Procédé selon l'une ou plusieurs des revendications 5 à 11, caractérisé en ce qu'il est détecté à partir de la distance (8,76) des postes de base et secondaires (6,73,7,74) et de la durée de propagation du rayonnement énergétique (10,79,83,87) et/ou du rayonnement énergétique de retour (12,14,81,85,89), en fonction de la vitesse de propagation du rayonnement, une distance (8,76) entre les postes de base et/ou secondaires (6,73,7,74) et l'objet mobile (2).

13. Procédé selon l'une ou plusieurs des revendications 2 à 12, caractérisé en ce que le code d'identification (30) est modulé sur le rayonnement énergétique (10,79,83,87), par exemple par une modulation de la fréquence.

14. Procédé selon l'une ou plusieurs des revendications 5 à 13, caractérisé en ce que le code d'idenfication (30) modulé sur le rayonnement énergétique (10,79,83,87) présente une impulsion de départ (53,56,127,130) respectivement un signal de départ pour commencer la détermination du temps aux postes de base (6,73) et secondaire (7,74,75) et/ou au point de référence, et que le rayonnement énergétique de retour (12,14,81,85,89) est déclenché par cette impulsion de départ (53,56,127,130).

15. Procédé selon l'une ou plusieurs des revendications 1 à 14, caractérisé en ce que sont émis par le rayonnement énergétique (10,79,83,87) à l'intérieur d'un laps de temps (57,131) du cycle de mesure maximal plusieurs, au moins deux codes d'identification (30) et le cas échéant des impulsions de départ (53,56,127,130) respectivement des signaux de départ et/ou des codes d'arrêt.

16. Procédé selon l'une ou plusieurs des revendications 1 à 15, caractérisé en ce qu'avec un code d'identification (30) plusieurs objets (2) respectivement les indicateurs de position (5,189,190) disposés sur les objets (2) sont appelés, et que les rayonnements énergétiques de retour (12,14,81,85,89,180) comprennent une fréquence de préférence différente (63,137) respectivement des codes d'identification différents (30) et/ou des instructions d'arrêt et/ou de départ.

17. Procédé selon l'une ou plusieurs des revendications 1 à 16, caractérisé en ce que la détermination des positions des différents objets (2) a lieu en temps réel.

**18.** Procédé selon l'une ou plusieurs des revendications 1 à 17, caractérisé en ce que les indicateurs de position (5,189,190) sont disposés sur différentes parties corporelles d'une personne (185) et/ou d'animaux, notamment au voisinage des articulations de ceux-ci.

**Fig.1**

Fig.6

Fig.2

Fig. 3

EP 0 740 801 B1

38

**Fig.4**

Fig. 5

**Fig.7**

Fig.8

**Fig.9**

**Fig.10**